# EUROPÄISCHE PATENTANMELDUNG

(11) **EP 1 223 216 A1**
(43) Veröffentlichungstag der Anmeldung: **17.07.2002**
(21) Anmeldenummer: 00128766.3
(22) Anmeldetag: 30.12.2000
(51) Int. Cl.: C12N 15/11, C12N 15/63, C12N 5/10, A01K 67/00, A61K 31/7088, A61K 48/00, G01N 33/53, C12Q 1/68

(54) **Intrazelluläre Nukleinsäureinhibitoren kleiner Guaninnukleotid-Austauschfaktoren**

(71) Anmelder: NascaCell GmbH, 83209 Prien (DE)
(72) Erfinder: Kolanus Waldemar, D-81245München (DE); Famulok Michael, D-53175 Bonn (DE); Blind Michael, D-81245 München (DE); Mayer Günter, D-53121 Bonn (DE)
(74) Vertreter: Bohmann, Armin K., Dr.

(57) **Zusammenfassung**

Die vorliegend Erfindung betrifft eine Nukleinsäure, insbesondere eine isolierte Nukleinsäure, die in der Lage ist, an einen Guaninnukleotid-Austauschfaktor für ADP-Ribosylierungsfaktoren zu binden, und deren Derivate.

## Beschreibung

Die vorliegende Erfindung betrifft Nukleinsäuren, die in der Lage sind, an einen Guaninaustauschfaktor zu binden, diese enthaltende Vektoren, diese enthaltende Zellen, Verwendungen derselben, diese enthaltende Zusammensetzungen, Inhibitoren für einen Guaninaustauschfaktor und Verfahren zum Screenen von Verbindungen, die die Wechselwirkung zwischen einem Guaninnukleotidaustauschfaktor und einer Nukleinsäure inhibieren.

Proteine der Familie der Guaninnukleotid-Austauschfaktoren (ARF-GEF's) für humane ADP-Ribosylierungsfaktoren (ARF's), die hierin auch als ARF-GEF's bezeichnet werden, wobei die ARF's wiederum kleine ras-ähnliche GTPasen sind, spielen eine kritische Rolle beim Transport intrazellulärer Vesikel und der Organisation des Aktinzytoskeletts. ARF's gehören zu einer großen Familie von monomeren G-Proteinen, die an einer Vielzahl von Signaltransduktionskaskaden und regulatorischen Prozessen beteiligt sind. Die ARF-Proteine von Säugern können in drei Klassen unterteilt werden: Klasse I (ARF 1, 2, 3), Klasse II (ARF 4 und 5) und Klasse III (ARF 6). ARF's interagieren mit einer Reihe regulatorischer Proteine (ARF-GEF's, GTPase aktivierende Proteine (GAPs), Arfaptine), Effektorproteinen (Coatomer, Phospholipase D, βγ-Untereinheiten von G-Proteinen), Choleratoxin, und Phospholipiden. Die Aktivität der ARF-Proteine wird durch die Art des gebundenen Nukleotidkofaktors bestimmt. GEF's, die die Freisetzung von gebundenen GDP und damit die Bindung von GTP fördern, aktivieren die ARF-Proteine. So wird der Austausch von hoch affin gebundenem GDP gegen GTP zur Aktivierung der ADP-Ribosylierungsfaktoren (ARF's) benötigt (Bourne et al., Nature 349 (1991), 117-127). Die Interaktion von Guaninnukleotidaustauschfaktoren (GEF's) mit dem ARF/GDP-Komplex erniedrigt die Affinität für GDP und erleichtert so dessen Dissoziation (Jackson. und Casanova, Trends Cell. Biol. 10 (2000), 60-67). Danach bindet GTP an die freie Nukleotidbindungsstelle des ARF/GEF-Komplexes, wodurch eine Konformationsänderung induziert wird, die zur Dissoziation des GEF's führt und den ARF/GTP-Komplex aktiviert (Peyroche et al., Mol. Cell 3 (1999), 275-285).

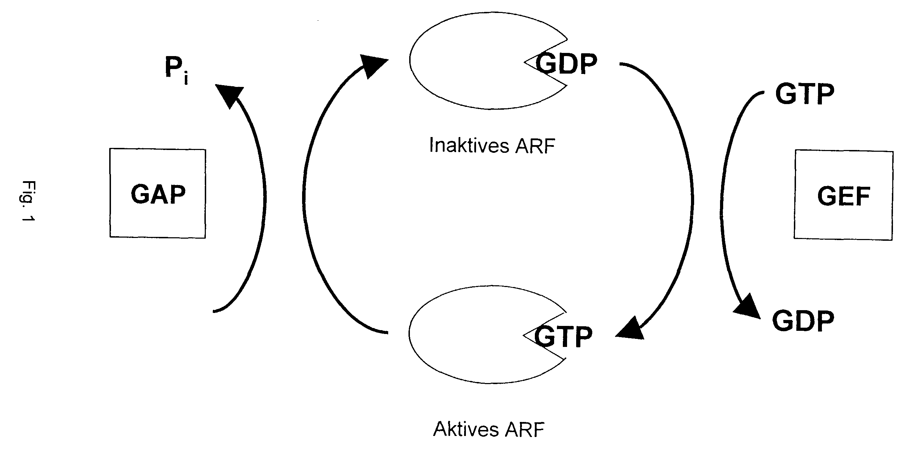

Die Inaktivierung der ARF-Proteine erfolgt durch die Hydrolyse des gebundenem GTP zu GDP, die von GTPase-aktivierenden Proteinen (GAPs) beschleunigt wird. Eine schematische Darstellung dieses Aktivierungs-/Deaktivierungszyklus ist in Fig. 1 dargestellt. ARF-GEF's können in zwei Familien unterteilt werden. Die großen oder hochmolekularen Guaninnukleotid-Austauschfaktoren (ARF-GEF's) mit einem Molekulargewicht über 100 kDa, unter anderem das Sec7 Protein aus der Hefe, Gea1, Gea2, BIG1 oder BIG2 zeichnen sich durch ihre charakteristische Sensitivität gegenüber dem niedermolekularen, organischen Inhibitor Brefeldin A aus. Brefeldin A bindet an den trimeren Intermediatkomplex von ARF, GDP und den großen ARF-GEF's, wodurch dieser stabilisiert wird. Dadurch wird der Austausch von GDP gegen GTP inhibiert und die Aktivierung der ARF-Proteine verhindert. Die großen ARF-GEF's spielen eine Rolle bei der Regulierung des endoplasmatischen Reticulums und des Golgiapparats. Die Zugabe von Brefeldin A führt zur Auflösung des Golgi und der Inhibition der Sekretion durch die Zellen. Demzufolge hat sich Brefeldin A als nützliche Substanz zur Untersuchung der Funktion der großen GEF's in vitro und in vivo erwiesen

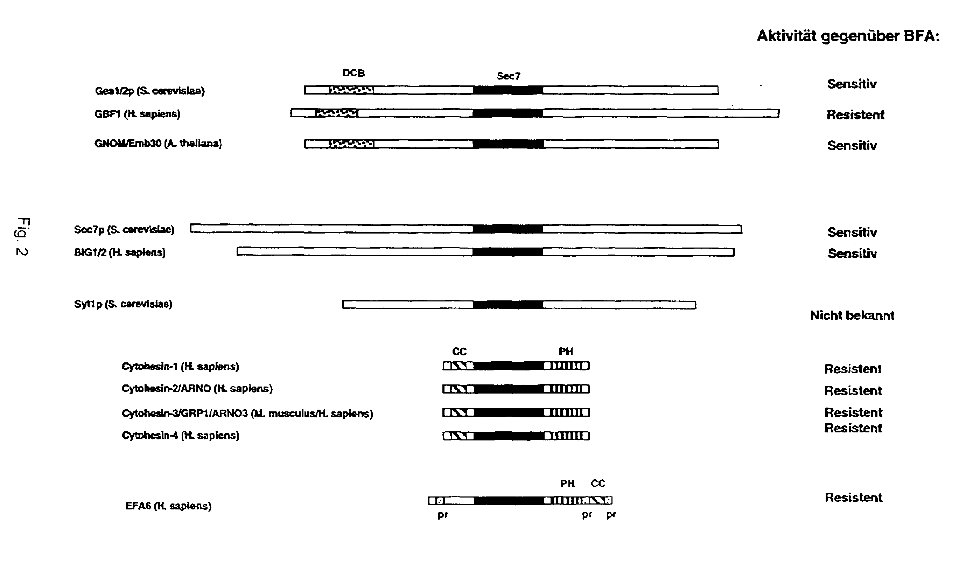

Die zweite Gruppe, zu der Cytohesin-1, Cytohesin-2 (ARNO), Cytohesin-3 (GRP-1/ARNO3) und Cytohesin-4 gehören, besitzt ein Molekulargewicht von ca. 50 kDa und wird nicht durch Brefeldin A inhibiert. Die kleinen ARF-GEF's sind hauptsächlich bei regulativen Vorgängen an dem endosomalen Kompertiment und an der Zytoplasmamembran beteiligt. Die ARF-GEF-Familie wurde unter anderem von Jackson und Casanova (Trends Cell. Biol. 10 (2000), 60-67) beschrieben und in Fig. 2 in einer Übersicht dargestellt (nach Donaldson und Jackson, Curr. Opin. Struct. Biol. 12 (2000), 475-482).. Ein gemeinsames Merkmal der ARF-GEF's ist ihre zentrale Sec7-Domäne; die weitgehend verantwortlich für die Katalyse des Guaninnukleotidaustauschs und die Sensitivität der großen GEF's gegenüber Brefeldin A ist.

Die kleinen ARF-GEF's sind impliziert in Signaltransduktionsprozessen, die über Pl 3-Kinasen vermittelt werden (Klarlund et al., Science 275 (1997), 1927-1930). Ferner sind sie beteiligt an der Reorganisation des Aktinzytoskeletts (Frank et al., Mol. Biol. Cell. 9 (1998), 3133-3146) und der Aktivierung von Integrinen (Geiger et al., EMBO J. 16 (2000), 3525-2536). Für die kleinen ARF-GEF's wurden bislang keine funktionellen Modulatoren oder Inhibitoren beschrieben, welche die direkte Untersuchung der Proteine in vitro und in Zellen ermöglichen. Daher wurden bislang die meisten Ergebnisse über ihre biologische Funktion durch Mutationsanalyse und die Expression/Überxpression der Proteine oder einzelner ihrer Domänen in Zellkultur gewonnen. Alle bislang identifizierten kleinen ARF-GEF's enthalten neben der SEC7-Domäne eine PH-("plecstrin homology")-Domäne, die die Lokalisation an Membranen durch Interaktion mit Polyphosphoinsitiden steuern kann. Daneben wird auch eine CC-("coiled coil")-Domäne gefunden, die vermutlich die Interaktion mit andere Proteinen vermittelt (vgl. Fig. 2 nach Donaldson und Jackson, Curr. Opin. Struct. Biol. 12 (2000), 475-482)

Der vorliegenden Erfindung liegt die Aufgabe zugrunde, einen Modulator für die Aktivität eines Guaninnukleotid-Austauschfaktors (ARF-GEF) für ARF-Proteine, insbesondere eines kleinen Guaninnukleotid-Austauschfaktors für ARF-Proteine bereitzustellen. Des weiteren liegt der Erfindung die Aufgabe zugrunde, einen Modulator für die GDP/GTP- Austauschfunktion eines Guaninnukleotid-Austauschfaktors, insbesondere eines kleinen Guaninnukleotid-Austauschfaktors bereitzustellen. Schließlich ist es eine weitere Aufgabe der vorliegenden Erfindung, ein Mittel bereitzustellen und Verfahren zur Herstellung derartiger Mittel, welches in den Prozess der durch Integrine vom Typ β-2 vermittelten Adhäsion von Immunzellen, insbesondere Leukozyten eingreift und somit ein Mittel darstellt, das bei der Therapie und Diagnose von Krankheiten, bei denen dieser Prozess beteiligt ist, eingreifen kann.

Erfindungsgemäß wird die Aufgabe gelöst durch eine Nukleinsäure, insbesondere eine isolierte Nukleinsäure, die in der Lage ist an einen Guaninnukleotid-Austauschfaktor für ADP-Ribosylierungsfaktoren zu binden und deren Derivate.

In einem zweiten Aspekt wird die Aufgabe gelöst durch eine Nukleinsäure, die in der Lage ist an einen Guaninnukleotid-Austauschfaktor für ADP-Ribosylierungsfaktoren zu binden, wobei die Nukleinsäure eine Sequenz umfaßt, die ausgewählt ist aus der Gruppe, die SEQ ID No. 1, SEQ ID No. 2, SEQ ID No. 3, SEQ ID No. 4, SEQ ID No. 5, SEQ ID No. 6, SEQ ID No. 7, SEQ ID No. 8, SEQ ID No. 9 und SEQ ID No. 10 sowie deren jeweilige Derivate umfaßt.

In einer Ausführungsform der erfindungsgemäßen Nukleinsäuren ist vorgesehen, dass die Derivate der Nukleinsäure wenigstens eine modifizierte Verbindungsgruppe, bevorzugterweise wenigstens eine modifizierte Verbindungsgruppe im Zuckerphosphatrückgrat und/oder wenigstens einen modifizierten Zuckerrest und/oder wenigstens eine modifizierte Base oder eine oder mehrere Kombinationen davon umfaßt.

In einer bevorzugten Ausführungsform der erfindungsgemäßen Nukleinsäuren ist vorgesehen, dass die modifizierte Base ausgewählt ist aus der Gruppe , die 5- Fluoruracil, 5-Bromuracil, 5-Chloruracil, 5-loduracil, Hypoxanthin, Xanthin, Ethenoadenosin, 4-Acetylcytosin, 5-(Carboxyhydroxylmethyl)uracil, 5- Carboxymethylaminomehtyl-2-thio-uridin, 5- Carboxymethylaminomethyluracil, Dihydrouracil, β-D-Galactosylqueosin, Inosin, N6-Isopentenyladenin, 1-Methyladenin, 1- Methylpseudouracil, 1-Methylguanin, 1-Methylinosin, 2,2-Dimethylguanin, 2-Methyladenin, 2-Ethylguanin, 3-Methylcytosin, 5-Methylcytosin, N6-Methyladenin, 7-Methylguanin, 5-Methylaminomethyluracil, 5-Methoxyaminomethyl-2-thiouracil, β-D-Mannosylqueosin, 5'-Methoxycarbonylmethyluracil, 5-Methoxyuracil, 2-Methylthio-N6-lsopentenyladenin, Uracil-5-oxyesseigsäuremethylester, Uracil-5-oxyessigsäure, Pseudouracil, Queosin, 3-Thiocytosin, 5-Methyl-2-thiouracil, 2-Thiouracil, 4-Thiouracil, 5-Methyluracil, Uracil-5-oxyessigsäuremethylester, Uracil-5-oxyessigsäure, 3(3-Amino-3-N-2-carboxypropyl)Uracil und 2,6-Diaminopurin.

In einer weiteren Ausführungsform der erfindungsgemäßen Nukleinsäuren ist vorgesehen , dass diese mindestens eine Modifikation oder Markierung am 5'- und/oder 3'-Ende, wobei bevorzugterweise die Modifikation und/oder die Markierung ausgewählt ist aus der Gruppe, die die Biotingruppe; die Digoxygeningruppe; Fluoreszenzfarbstoffe, insbesondere Fluorescein und Rhodamin; Psoralen; Thiolgruppe(n), Aminogruppe(n), Ethylenglykolgruppe(n)- oder Cholesterylgruppe(n) enthält.

In einer noch weiteren Ausführungsform der erfindungsgemäßen Nukleinsäuren ist vorgesehen, dass die modifizierte Verbindungsgruppe ausgewählt ist aus der Gruppe, die Phosphomono- oder Phosphodithioate, Alkylphosphonate, Arylphosphonate, Phosphoroamidate, Phosphattriester, bevorzugterweise P(O)-Alkylderivate; Verbindungsgruppen, in denen Sauerstoffatome in der durch die Phosphatgruppe gebildetet Brücke zwischen den Zuckern durch andere Bindungen ersetzt werden, bevorzugterweise NH-, CH₂- oder S-P-Verbindungen, bevorzugtererweise 3'-NHP(O)-(O⁻)O-5'phosphoramidate; Dephosphointernukleotidverbindungen, bevorzugterweise Acetamidat-, Carbamatverbindungen oder Peptidnukleinsäuren (PNA) umfaßt.

In einer Ausführungsform der erfindungsgemäßen Nukleinsäuren ist vorgesehen, dass der modifizierte Zuckerrest ausgewählt ist aus der Gruppe, die 2'-Azido-2'-Deoxy-, 2'-Amino-2'-Deoxy-, 2'-Fluoro-2'-Deoxy-, 2'-Chloro-2'-Deoxy, 2'-O-Methyl, 2'-O-Allyl-, 2'-C-Fluoromethyl-Gruppen und/oder Modifikationen umfaßt.

In einer Ausführungsform der erfindungsgemäßen Nukleinsäuren ist vorgesehen, dass der Guaninnukleotidaustauschfaktor zur Gruppe der kleinen Guaninnukleotidaustauschfaktoren für ARF-Proteine gehört, insbesondere zur Gruppe der Guaninnukleotidaustauschfaktoren, deren Molekulargewicht etwa 50 kDa oder weniger beträgt.

In einer noch weiteren Ausführungsform der erfindungsgemäßen Nukleinsäuren ist vorgesehen, der Guaninnukleotidaustauschfaktor nicht durch Brefeldin A inhibiert wird.

In einer bevorzugten Ausführungsform der erfindungsgemäßen Nukleinsäuren ist vorgesehen, dass der Guaninnukleotidaustauschfaktor Cytohesin-1 oder Cytohesin-2 und insbesondere die Sec7-Domäne von Cytohesin-1 oder Cytohesin-2 ist.

In einer weiteren bevorzugten Ausführungsform der erfindungsgemäßen Nukleinsäuren ist vorgesehen, dass der Guaninnukleotidaustauschfaktor die Sec7-Domäne ist.

In einer noch weiteren Ausführungsform der erfindungsgemäßen Nukleinsäuren ist vorgesehen, dass die Nukleinsäure eine solche ist, die ausgewählt ist aus der Gruppe, die DNA, RNA, Polynukleotide, Oligonukleotide, Aptamer, Aptazyme und Intramere umfaßt.

In einem dritten Aspekt wird die Aufgabe gelöst durch einen Vektor umfassend eine erfindungsgemäße Nukleinsäure.

In einer Ausführungsform ist vorgesehen, dass der Vektor ein Expressionsvektor ist.

In einem vierten Aspekt betrifft die Erfindung eine Zelle umfassend eine erfindungsgemäße Nukleinsäure und/oder einen erfindungsgemäßen Vektor.

In einer Ausführungsform ist vorgesehen, dass die Zelle eine eukaryontische Zelle, bevorzugterweise eine tierische Zelle und ganz bevorzugt eine Zelle eines Säugetiers ist.

In einer weiteren Ausführungsform ist vorgesehen, dass die Zelle ausgewählt ist aus der Gruppe, die Saccharomyces cerevisiae und C. elegans umfasst.

In einer noch weiteren Ausführungsform der erfindungsgemäßen Zelle ist vorgesehen, dass das Säugetier ausgewählt ist aus der Gruppe, die Mäuse, Ratten, Kaninchen, Hunde, Schweine, Affen und Menschen umfasst.

In einem fünften Aspekt betrifft die Erfindung ein Tier, bevorzugterweise ein transgenes Tier, das mindestens eine erfindungsgemäße Zelle umfasst.

In einer Ausführungsform ist vorgesehen, dass das Tier ausgewählt ist aus der Gruppe, die Nematoden, Fische, Mäuse, Ratten, Kaninchen, Hunde, Schweine und Affen umfasst.

In einem sechsten Aspekt betrifft die Erfindung die Verwendung einer erfindungsgemäßen Nukleinsäure zur Herstellung eines Medikamentes.

In einer Ausführungsform ist, dass das Medikament für die Behandlung von Erkrankungen ist, die ausgewählt sind aus der Gruppe, die Metastasierung von Lymphomen oder Melanomen, Autoimmunerkrankungen, Abstoßungsreaktionen, akute und chronische Entzündungen, Reperfusionsschäden, Transplantationserkrankungen, insbesondere Abstoßungsreaktionen bei Organtransplantationen und graft-vs-host-Erkrankungen umfasst.

In einer Ausführungsform der erfindungsgemäßen Nukleinsäuren ist vorgesehen, dass das Medikament die durch β-2 - Integrine vermittelte Adhäsion von Immunzellen beeinflußt.

In einem siebten Aspekt betrifft die Erfindung die Verwendung einer erfindungsgemäßen Nukleinsäure und/oder eines erfindungsgemäßen Vektors in der Gentherapie.

In einem achten Aspekt betrifft die Erfindung die Verwendung einer erfindungsgemäßen Nukleinsäure zum Nachweis eines Guaninnukleotidaustauschfaktors

In einem neunten Aspekt betrifft die Erfindung die Verwendung einer erfindungsgemäßen Nukleinsäure zur Komplexbildung mit einem Guaninnukleotidaustauschfaktor.

In einem zehnten Aspekt betrifft die Erfindung eine Zusammensetzung, insbesondere pharmazeutische Zusammensetzung, umfassend eine erfindungsgemäße Nukleinsäure, einen erfindungsgemäßen Vektor und/oder eine erfindungsgemäße Zelle zusammen mit einem geeigneten Trägermaterial, insbesondere einem pharmazeutisch akzeptablen Träger.

In einem elften Aspekt betrifft die Erfindung einen Inhibitor für einen Guaninnukleotidaustauschfaktor, wobei der Guaninnukleotidaustauschfaktor zur Gruppe der kleinen Guaninnukleotidaustauschfaktoren für ARF-Proteine gehört, insbesondere zur Gruppe der Guaninnukleotidaustauschfaktoren, deren Molekulargewicht etwa 50 kDa oder weniger beträgt.

In einer Ausführungsform ist vorgesehen, dass der Guaninnukleotidaustauschfaktor nicht durch Brefeldin A inhibiert wird.

In einer weiteren Ausführungsform ist vorgesehen, dass der Inhibitor eine erfindungsgemäße Nukleinsäure und/oder einen erfindungsgemäßen Vektor umfaßt.

In einem zwölften Aspekt betrifft die Erfindung ein Verfahren zum Screenen von Verbindungen, die die Wechselwirkung zwischen einem Guaninnukleotid-Austauschfaktor und einer erfindungsgemäßen Nukleinsäure inhibieren, insbesondere durch ein Verfahren, das mit Hochdurchsatzverfahren kompatibel ist und durch folgende Schritte gekennzeichnet ist:
a) Bereitstellen des Guaninnukleotid-Austauschfaktors und der Nukleinsäure
b) optional Bestimmen, ob eine Wechselwirkung zwischen dem Guaninnukleotid-Austauschfaktor und dem β-2-Integrin erfolgt,
c) Hinzufügen einer Kandidaten-Verbindung , und
d) Bestimmen, ob eine Wechselwirkung zwischen dem Guaninnukleotid-Austauschfaktor und der Nukleinsäure, bevorzugterweise durch die Kandidaten-Verbindung, inhibiert wird.

In einer Ausführungsform ist vorgesehen, dass als weiterer Schritt vorgesehen ist Bestimmen, ob die identifizierte Verbindung die Guaninnukleotid-Austauschfunktion des Guaninnukleotid-Austauschfaktors für ein monomeres G-Protein inhibiert.

In einer weiteren Ausführungsform ist vorgesehen, dass als weiterer Schritt vorgesehen ist Bestimmen, ob die Interaktion eines Guaninnukleotid-Austauschfaktors mit einem Integrin, bevorzugterweise die Wechselwirkung zwischen der Sec-7-Domäne von Cytohesin-1 und der β-2-Integrinuntereinheit inhibiert wird.

In einer alternativen Ausführungsform ist vorgesehen, dass als weiterer Schritt vorgesehen ist Bestimmen, ob die Interaktion der PH-Domäne mit ihren natürlichen Liganden inhibiert wird, bevorzugterweise die Interaktion der PH-Domäne von Cytohesin-1 mit Phosphatidylinositol-3,4,5-trisphosphat inhibiert wird.

In einer weiteren Ausführungsform des erfindungsgemäßen Verfahrens ist vorgesehen, dass die Kandidaten-Substanz zur Herstellung eines Medikamentes verwendet wird.

In einer bevorzugten Ausführungsform ist, dass das Medikament für die Behandlung von Erkrankungen ist, die ausgewählt sind aus der Gruppe, die Metastasierung von Lymphomen oder Melanomen, Autoimmunerkrankungen, Abstoßungsreaktionen, akute und chronische Entzündungen, Reperfusionsschäden, Transplantationserkrankungen, insbesondere Abstoßungsreaktionen bei Organtransplantationen und graft-vs-host-Erkrankungen umfasst

In einer weiteren Ausführungsform ist vorgesehen, dass die erfindungsgemäße Nukleinsäure, der Guaninnukleotid-Austauschfaktor oder beide mit Fluoreszenzfarbstoffen markiert sind und die Inhibition der Wechselwirkung zwischen der Nukleinsäure und dem Guaninnukleotid-Austauschfaktor zu einer meßbaren Veränderung des Fluoreszenzsignals führt.

In einer noch weiteren Ausführungsform ist vorgesehen, dass die Nukleinsäure oder der Guaninnukleotid-Austauschfaktor als Wechselwirkungspartner mit einer fluoreszenzlöschenden Gruppe (Quencher), der jeweils andere Wechselwirkungspartner mit einer fluorophoren Gruppe (Donor) markiert ist, wobei die Wechselwirkung zwischen beiden Wechselwirkungspartnern zur Löschung der Fluoreszenzemission des Donors führt, und die Inhibition der Wechselwirkung zur Freisetzung der Fluoreszenz des Donors führt.

In einer Ausführungsform ist vorgesehen, dass die Nukleinsäure ein Aptazym ist und die Inhibition der Bindung des Guaninnukleotid-Austauschfaktors an das Aptazym durch eine Veränderung der katalytischen Aktivität des Aptazyms gemessen werden kann.

In einer noch weiteren Ausführungsform ist, dass die Wechselwirkung zwischen den Wechselwirkungspartner erfindungsgemäße Nukleinsäure und Guaninnukleotid-Austauschfaktor mittels Detektionsverfahren wie enzymbasierenden Assays surface plasmon resonance (SRP), fluorescence activated cell sortting FACS, fiberoptische Microarray Sensoren, Kapillarelektrophorese, quartz crystal microbalance (QCM) oder radioaktiven Messverfahren erfolgt.

In einem dreizehnten Aspekt betrifft die Erfindung die Verwendung eines Guaninnukleotid-Austauschfaktors als Zielmolekül im Rahmen eines in vitro Selektions-Verfahrens.

In einem vierzehnten Aspekt betrifft die Erfindung ein Verfahren zur Identifizierung und Isolierung von Nukleinsäuren, die in der Lage sind, an ein Zielmolekül zu binden, das durch die folgenden Schritte gekennzeichnet ist:
- Inkubieren des Zielmoleküls oder eines Teils davon mit einer Vielzahl von Nukleinsäuren, bevorzugterweise einer Nukleinsäurebibliothek, wobei die Nukleinsäuren verschiedene Sequenzen aufweisen,
- Selektieren und Isolieren jener Nukleinsäuren, die in der Lage sind, an das Zielmolekül oder einen Teil davon zu binden,
- optional Amplifizieren der isolierten Nukleinsäuren and Wiederholen der ersten beiden Schritte; und
- optional Bestimmen der Sequenz und/oder Bindungsspezifität der isolierten Nukleinsäuren,
wobei, dass das Zielmolekül ein Guaninnukleotid-Austauschfaktor, bevorzugterweise ein solcher für ARF ist.

In einem fünfzehnten Aspekt betrifft die Erfindung die Verwendung einer erfindungsgemäßen Nukleinsäure, wobei die Nukleinsäure bevorzugterweise in einem Komplex mit einem Guaninnukleotid-Austauschfaktor vorliegt, zum rationalen Design von Verbindungen. Bei den Verbindungen handelt es sich bevorzugterweise um Inhibitoren und bevorzugtererweisen um niedermolekularen Inhibitoren. Im Zusammenhang damit dient die Struktur der erfindungsgemäßen Nukleinsäuren als Leitstruktur für niedermolekulare oder kleine Verbindungen, die wie der erfindungsgemäßen Nukleinsäuren inhibierend wirken (können).

Bei dem erfindungsgemäßen Verfahren zum Screenen von Verbindungen, die die Wechselwirkung zwischen einem Guaninnukleotidaustauschfaktor und einer erfindungsgemäßen Nukleinsäure inhibieren, kann vorgesehen sein, dass die Kandidaten-Verbindung eine beliebige Verbindung sein, die beispielsweise aus einer Verbindungsbibliothek stammt, deren Fähigkeit getestet werden soll, ob sie die besagte Wechselwirkung zu beeinflussen, insbesondere zu inhibieren in der Lage ist.

Bei dem erfindungsgemäßen Verfahren zum Screenen von Verbindungen, die die Wechselwirkung zwischen einem Guaninnukleotidaustauschfaktor und einer erfindungsgemäßen Nukleinsäure inhibieren, kann das Detektionsverfahren auf enzymbasierenden Assays analog zu auf Antikörper basierenden ELISAs beruhen.

Bei der erfindungsgemäßen Verwendung eines Guaninnukleotid-Austauschfaktors als Zielmolekül im Rahmen eines in vitro Selektions-Verfahrens ist vorgesehen, dass der ARF-GEF ein jeder ARF-Gef ist, an den auch die erfindungsgemäßen Nukleinsäuren binden.

Der Erfindung liegt die überraschende Erkenntnis zu Grunde, dass Nukleinsäuren oder Nukleinsäureliganden gegen die kleinen ARF-GEF-Proteine erzeugt werden können, die, im Komplex mit ihrem jeweiligen Target, d.h. einem Guaninnukleotid-Austauschfaktor oder einem Teil, insbesondere einer Domäne davon, eine oder mehrere Funktionen bzw. Aktivitäten modulieren. Typischerweise handelt es sich bei der Modulierung der Funktion bzw. der Aktivität um eine Inhibierung. Die jeweilige Funktion oder Aktivität ist dabei typischerweise in einer oder mehreren der den Guaninaustauschfaktor aufbauenden Domänen lokalisiert. Ein Beispiel für eine Aktivität, die durch die erfindungsgemäßen Nukleinsäuren inhibiert wird, ist die GDP/GTP-Austauschaktivität des Guaninnukleotid-Austauschfaktors. Bei dem Guaninnukleotidautauschfaktor handelt es sich bevorzugterweise um einen solchen für humane ADP-Ribosylierungsfaktoren.

Die erfindungsgemäßen Nukleinsäuren sind bevorzugt isolierte Nukleinsäuren. Unter dem Begriff Nukleinsäuren sollen hierin auch Oligonukleotide und Polynukleotide verstanden werden. Der Begriff isolierte Nukleinsäure soll hierin insbesondere solche Nukleinsäuren bezeichnen, die im wesentlichen frei sind von Nukleinsäuren mit einer anderen Nukleinsäuresequenz. Derartige isolierte Nukleinsäuren sind beispielsweise solche, die durch chemische oder enzymatische Synthese hergestellt sind.

Die inhibierende Wirkung der erfindungsgemäßen Nukleinsäuren kann dabei sowohl in vitro nachgewiesen oder verwendet werden, wie auch in situ, d.h. in lebenden Zellen und auch in einem diese Zellen umfassenden Organismus. Die Verwendung kann dabei zu diagnostischen wie auch zu therapeutischen Zwecken erfolgen. Dies konnte in dieser Form bislang nach dem Stand der Technik durch keine andere Molekülklasse, wie Antikörper, Peptide, Oligosaccharide oder niedermolekulare Substanzen, insbesondere solchen, die aus kombinatorischen Bibliotheken isoliert worden sind, bewerkstelligt werden.

Wie oben bereits ausgeführt, werden mit den hierin offenbarten Nukleinsäuren Inhibitoren von Guaninnukleotid-Austauschfaktoren bereitgestellt, wobei beachtlich ist, dass mit den erfindungsgemäßen Nukleinsäuren erstmals Inhibitoren für die kleinen ARF-GEF's bereitgestellt werden. Bei diesen Inhibitoren handelt es sich somit typischerweise bzw. bevorzugterweise um exogene und/oder vergleichsweise kleine Moleküle, die verschieden sind von denjenigen Molekülen oder Derivaten oder Mutanten davon, mit denen der Guaninnukleotidaustauschfaktor in situ in Wechselwirkung tritt. Bei letzteren handelt es sich in der Regel um Proteine und insbesondere um einen ADP-Ribosylierungsfaktor bzw. ADP-Ribosylierungsfaktoren. Insbesondere sollen hierin unter Inhibitoren nicht solche verstanden werden, die beispielsweise aus dem Guaninnukleotidaustauschfaktor selbst, mutierten Varianten des Guaninnukleotidaustauschfaktors, Teilen des Guaninnukleotidaustauschfaktors, in situ intergierenden Proteinen wie den ARF-Proteinen, mutierten Varianten von in situ intergierenden Proteinen oder Teilen von in situ intergierenden Proteinen bestehen. Solche Proteinkonstrukte können wie die isolierte PH-Domäne von Cytohesin-1 als transdominant negative Inhibitoren eingesetzt werden. Die Verwendung der isolierten isolierten PH-Domäne als transdominant negativer Inhibitor von Cytohesin-1 ist hierin und in Nagel W. et al. (J. Biol. Chem. 9 (1998), 14853-14861) beschrieben. Die Inhibition beruht bei diesen Methoden beispielsweise auf der Komplexierung eines natürlichen Interaktors der Guaninnukleotidaustauschfaktoren in situ oder der Komplexierung des endogenen Guaninnukleotidaustauschfaktors selbst. Dabei ist es jedoch auch im Umfang der vorliegenden Erfindung, dass die Nukleinsäure, die erfindungsgemäß als Inhibitor wirkt, als solches oder in derivatisierter Form bereits Teil des Transkriptoms ist. Unter Transkriptom wird hierin die Gesamtheit aller transkribierten Ribonukleinsäuren einer Zelle bzw. eines Organismus verstanden. Mit anderen Worten, es ist im Rahmen der vorliegenden Erfindung, dass die erfindungsgemäße Nukleinsäure und damit auch der erfindungsgemäße Inhibitor identisch ist mit einer Nukleinsäuresequenz, die natürlicherweise in einer Zelle vorhanden ist, die jedoch nicht notwendigerweise an kleine ARF-GEF's bindet, was zum verschiedene Ursachen haben kann. Eine derartige mögliche Ursache kann die Tatsache sein, dass die in der Zelle vorkommende Sequenz an einer anderem Stelle oder in einem anderen Kompartiment der Zelle lokalisiert ist als die ARF-GEF's. Eine andere mögliche Ursache kann der verschiedene Zeitpunkt der Expression des ARF-GEF's relativ zu derjenigen der besagten Nukleinsäure sei. Eine noch weitere Ursache kann darin bestehen, dass die Menge der Nukleinsäure nicht ausreicht, um an die ARF-GEF's in einem Umfang zu binden, dass durch das Bindungsereignis eine Funktion der ARF-GEF's beeinträchtigt wird.

Ohne im folgenden darauf festgelegt sein zu wollen, scheint den hierin offenbarten vielfältigen medizinischen Verwendungen der erfindungsgemäßen Nukleinsäure die folgende Wechselwirkung zugrunde zu liegen. Dieser Mechanismus ist anhand von Cytohesin, insbesondere Cytohesin-1 beschrieben, gilt jedoch grundsätzlich für alle hierin beschriebenen Guaninnukleotid-Austauschfaktoren

Cytohesin-1 spielt nach dem derzeitigen Verständnis eine kritische Rolle bei der Steuerung der Adhäsion von Leukozyten und der Kontrolle der dynamischen Regulierung des Zytoskeletts. Cytohesin-1, ein zytoplasmatisches 47 kDa Protein, wird spezifisch in Leukozyten des Immunsystems gebildet (Kolanus, W., et al., Cell 86 (1996), 233-242). Dieses regulatorische Protein besteht aus einer aminoterminalen Coiled-Coil-Struktur, einer zentralen Sec7-Domäne, und einer carboxyterminalen PH-("plecstrin homology")-Domäne, an die eine polybasische C-Domäne angrenzt. Cytohesin-1 ist ein Schlüsselregulator der auf Leukozyten vorkommenden β2-Integrine. Cytohesin-1 aktiviert durch Interaktion mit der zytoplasmatischen Domäne der β2-Untereinheit die Adhäsion der extrazellulären Domänen des Integrins LFA-1 (αL/β2; CD11a/CD18) an seinen spezifischen Liganden ICAM-1 ("intercellular adhesion molecule 1") (Kolanus und Zeitimann, Curr. Top. Microbiol. Immunol. 231 (1998), 33-49). Die Interaktion zwischen Cytohesin-1 und der intrazytoplasmatischen Domäne des β-2-Integrins konnte durch Two Hybrid Experimente nachgewiesen werden (Kolanus, et al., Cell 86 (1996), 233-242). Dabei wird Cytohesin-1 durch kooperative Interaktion des polybasischen C-Peptids und der PH-Domäne mit dem Membranphospholipid Phosphatidylinositol-3,4,5-trisphosphat, das nach Stimulierung der Leukozyten z. B. durch den T-Zellrezeptor von dem Enzym Phosphoinositid 3-Kinase gebildet wird, an die Zytoplasmamembran rekrutiert (Nagel, et al., J. Biol. Chem. 9 (1998), 14853-14861; Venkateswarlu, et al., J. Cell. Sci. 112 (1999), 1957-1965). Dort führt die Interaktion der Sec7-Domäne von Cytohesin-1 mit der β2-lntegrin-Untereinheit zur Aktivierung der β2-lntegrinrezeptoren (Kolanus, et al., Cell 86 (1996), 233-242). Überexpression von Cytohesin-1 oder Subdomänenkonstrukten der Sec7-Domäne in einer Jurkat E6 Zelllinie führt zur konstitutiven Adhäsion des LFA-1-Integrins (αL/β2) an ICAM-1. Wird dagegen die isolierte PH-Domäne von Cytohesin-1 in Leukozyten überexprimiert können die Zellen nach einem Stimulus nicht mehr an ICAM-1 adhärieren. Dieser dominant negative Effekt kann für eine Mutante der PH-Domäne (R281C), in der Arginin an Position 281 gegen Cystein ausgetauscht wurde, nicht beobachtet werden (Nagel, W. et al., J. Biol. Chem. 9 (1998), 14853-14861). Erklärt wird dieser Effekt mit der Blockierung der Interaktion des endogenen Cytohesin-1 mit Phosphatidylinositol-3,4,5-trisphosphat durch die isolierten, überexprimierten PH-Domänen. Dadurch wird die Rekrutierung von Cytohesin-1 an die Cytoplasmamembran und die darauffolgende Aktivierung des LFA-1-Integrins verhindert. Die erfindungsgemäßen Nukleinsäuren können die Assoziation mit der Zytoplasmamembran in analoger Weise durch die direkte Inhibition der PH-Domäne des endogenen Cytohesins inhibieren.

Eine weitere Funktion konnte in Zellkulturstudien der GDP/GTP-Austauschfunktion der Sec7-Domäne von Cytohesin-1 in Zusammenhang gebracht werden. Eine mutante Form von Cytohesin-1, bei der durch eine Punktmutation an Position 157 Glutamat gegen Lysin ausgetauscht wurde (Mutante E157K). Die Mutante E157K kann in vitro den GDP/GTP-Austausch nicht mehr katalysieren. Es wurde gefunden, dass die Überexpression von E157K die Adhäsion von Leukozyten an mit ICAM-1 beschichtete Oberflächen inhibiert und den morphologischen Phänotyp beeinflusst. Trotzdem wurde gefunden, dass sowohl bei der Überexpression von Cytohesin-1 als auch E157K ein mit der Aktivierung des LFA-1-Integrins korrespndierendes Anitkörperepitop induziert wird (Geiger et al., C., EMBO J. 16 (2000), 3525-2536). Der das Aktivierungsepitop erkennende Antikörper mAb24 wurde von Stewart und Mitarbeitern (Stewart et al., J Immunol 156 (1996), 1870-1817) beschrieben. Wie in der vorliegenden Erfindung in den Beispielen beschrieben, inhibieren die erfindungsgemäßen Nukleinsäuren und die Mutante E157K die Restrukturierung des Zytoskeletts. Nach dem derzeitigen Stand der Technik greift die Sec-7-Domäne von Cytohesin-1 in zwei separate Schritte der Adhäsion von Leukozyten ein, die beide für die firme Anheftung der Zellen an Oberfläche benötigt werden: i) Aktivierung der β-2-Integrine und ii) darauffolgende Restrukturierung des Zytoskeletts. Die erfindungsgemäßen Nukleinsäuren blockieren beide Mechanismen, wobei der zweite Mechanismus, der von der ARF-GEF-Aktivität abhängige Mechanismus in den Beispielen weiter beschrieben ist.

Infolge dieser spezifischen Inhibition der ARF-GEF-Aktivität von Cytohesin-1 und der Inhibition der Adhäsion von Leukozyten an Oberflächen bieten sich eine Vielzahl von möglichen Anwendungen der erfindungsgemäßen Nukleinsäuren, insbesondere auch im medizinischen Bereich, die letztlich auf der Blockierung dieser Wechselwirkung durch die erfindungsgemäßen Nukleinsäuren beruht. Weiterhin ergibt sich aus der vorstehend geschilderten Spezifität auch die Übertragbarkeit der verschiedenen Anwendungen auch auf solche Guaninnukleotid-Austauschfaktoren, die ebenfalls eine Sec7-Domäne oder eine dazu homologe Domäne besitzen, und damit auf die Gruppe der kleinen GEF's mit einem Molekulargewicht von etwa 50 kDa bzw den nicht durch Brefeldin A inhibierbaren GEF's., zu denen, u.a. neben Cytohesin-1 auch Cytohesin-2, Cytohesin-3 und Cytohesin-4 gehören.

Cytohesin-2 (ARNO) kann in vitro den GDP/GTP-Austausch von ARF-1 und ARF-6 katalysieren, lokalisiert an der Zytoplasmamamembran und hat Einfluss auf die Strukturierung des Aktinzytoskeletts (Frank, S.R. et al., Mol. Biol. Cell 9 (1998), 3133-3146; Venkateswarlu K. et al., Curr Biol 8 (1998), 463-466). Cytohesin-3 (GRP1/ARNO3) katalysiert den GDP/GTP-Austausch von ARF-1 in vitro, ARF6 in vivo und wird mit der Kontrolle der Organisation des Golgi-Apparates in Zusammenhang gebracht (Franco, M. et al., Proc. Natl. Acad. Sci USA 95 (1998), 9926-9931; Langille et al., J. Biol. Chem. 274 (1999), 27099-27104). Ferner wurde gefunden, dass Cytohesin-3 selektiv in anergen T-Helferzellen (Th1) induziert wird (Korthauer et al., J. Immunol. 164 (2000), 308-318). Das neueste Mitglied der kleinen ARF-GEF-Familie, Cytohesin-4, wird hauptsächlich in Leukozyten, aber nicht in den meisten anderen Geweben gefunden (Ogasawara et al., J. Biol. Chem. 275 (2000), 3221-3230). Die erfindungsgemäßen Nukleinsäuren können auch im Zusammenhang mit diesen Cytohesinen verwendet werden, wobei die Verwendung in der Inhibierung der Funktion, insbesondere der vorstehend genannten Funktion(en) der jeweiligen Cytohesine, besteht.

Die möglichen Anwendungen der erfindungsgemäßen Nukleinsäuren, insbesondere im Bereich der Medizin, ergeben sich auch aus der Art der Beteiligung der durch β-2-Integrine vermittelten Adhäsion an den verschiedenen Krankheitsbildern bzw. Pathogenitätsmechanismen. Wie bereits oben ausgeführt, kann durch die Blockierung der Bindungseigenschaften der ARF-GEF's, insbesondere deren Sec7-Domänen vermittels der erfindungsgemäßen Nukleinsäuren die durch β-2-Integrine vermittelte Adhäsion der Leukozyten verhindert werden. Demzufolge können die erfindungsgemäßen Nukleinsäuren verwendet werden zur Behandlung von Krankheiten, die auf der Adhäsion der Leukozyten beruhen, wie im folgenden ausgeführt. Außerdem kann unter Verwendung der erfindungsgemäßen Nukleinsäuren ein Screening-System aufgebaut werden, bei dem auf Verbindungen, insbesondere kleine Verbindungen (engl. small molecules) gescreent wird, die ebenfalls eine inhibitorische Wirkung vergleichbar derjenigen der erfindungsgemäßen Nukleinsäuren aufweisen.

Den exklusiv auf Leukozyten vorkommenden β-2-Integrinen kommt eine essentielle Funktion bei der regulierten Adhäsion von Immunzellen zu. Diese Prozesse steuern unter anderem die Zytotoxizität von T-Lymphozyten und NK-("natural killer")-Zellen, die Migration von Leukozyten zu entzündeten Geweben, die Phagozytose und das chemotaktische Verhalten von myeloiden Zellen. Charakteristisch für Integrine ist die Regulierbarkeit der Bindung an ihre spezifischen Liganden. Die heterodimeren Integrine bestehen aus einer α- und einer β-Untereinheit. Jede β-Kette ist in der Lage, mit verschiedenen α-Untereinheiten zu paaren, und so mehrere Rezeptoren mit individuellen Spezifitäten zu bilden. Je nach der in dem Dimer gefundenen β-Untereinheit werden die Integrine in mehrere Unterfamilien eingeteilt. Bisher wurden in Vertebraten 16 verschiedene α-Untereinheiten und 8 verschiedene β-Untereinheiten identifiziert. Da der durch β2-Integrine vermittelten Adhäsion von Immunzellen eine zentrale Bedeutung bei der Regulierung des Immunsystems zukommet, stellt die Inhibition dieses Ereignisses den Ansatzpunkt für eine therapeutische Intervention bei klinischen Indikationen wie akuten Entzündungen, Reperfusionsschäden oder der Organtransplantation (Schürmann, Chirurg 68 (1997), 477-487). Ohne auf die nachfolgend beschriebenen Beispiele festgelegt zu sein, konnte bei Reperfusionsschäden des Myokard (Jones S. P. et al., Am J Physiol Heart Circ Physiol 279 (2000), H2196-2201; Lefer A. M., Ann Thorac Surg 68 (1999), 1920-1923), der Niere (Tajra L. C. et al., J Surg Res 87 (1999), 32-38) oder der cerebralen Blutgefäße (Prestigiacomo C.J. et al., Stroke 30 (1999), 1110-1117) eine Verminderung der Gewebeschädigungen durch Reduktion der β-2-Integrinaktivität nachgewiesen werden. Damit eröffnet sich ein breites therapeutisches Potential für die Inhibition der durch β-2-Integrine vermittelten Adhäsion von Leukozyten in der Transplantationsmedizin oder der Behandlung von Herzinfarkten oder Schlaganfällen.

Die Blockierung der Adhäsion von Leukozyten über β-2-Integrine vermittels der erfindungsgemäßen Nukleinsäuren kann auch ein therapeutischer Ansatzpunkt für die Behandlung von akuten oder chronischen Entzündungen sein. Als nicht limitierende Beispiele seien die rheumatoide Arthritis (Issekutz A.C., Inflamm Res 47 Suppl 3 (1998), 123-132; Torrsteinsdottir I. et al., Clin Exp Immunol 115 (1999), 554-560) oder entzündlichen Erkrankungen der Haut wie Psoriasis (Schon et al., J Invest Dermatol 114 (2000), 976-983) genannt. Blockade der Adhäsivität von Leukozyten könnte hier zu einer Reduktion der inflammatorischen Prozesse und einer Verbesserung der klinischen Symptome führen.

Ebenso wird Integrinen eine Rolle bei der Metastasierung von Tumoren, z.B. bei Lymphomen, Leukämien oder Melanomen zugeschrieben (Giancotti, F. G. und Mainiero, F., Biochim Biophys Acta 1198 (1994), 47-64). Ein entscheidender Schritt in diesem Prozess ist die Anheftung der im Blutstrom zirkulierenden, transformierten Zellen an die Zielgewebe. Dieses Steuerung kann durch Adhäsionsmoleküle auf den Oberflächen der Zellen vermittelt werden. Es konnte beispielsweise gezeigt werden, dass Lymphomzellen mit hoher LFA-1 Expression mehr zur Metastasierung neigen als Zellen mit niedrigeren Expressionsniveaus. In diesem Fall könnte die Blockierung der Adhäsion das metastatische Potential der Lymphomzellen erniedrigen, wie auch schon durch die Blockierung der extrazellulären β-2-Integrinuntereinheit mit Antikörpern gezeigt werden konnte (Zahalka M. A. et al., J Immunol 150 (1993), 4466-4477). Ebenso wird ein Zusammenhang zwischen veränderter β-2-Integrinexpression und dem metastatischen Potential bei anderen Krebsarten, wie Leukämien (Puig-Kroger A. et al., J. Biol Chem 275 (2000), 28507-28512) oder Melaomen (Menter D.G., Immunol Cell Biol 73 (1995), 575-583) vermutet. Es liegt daher nahe, dass durch Inhibition der Anheftung der disseminierenden Zellen könnte bei bestimmten Tumorarten das metastatische Potential gesenkt werden könnte.

Ein weiterer Aspekt der Erfindung betrifft ein Verfahren, die sogenannte in vitro Selektion, mit deren Hilfe spezifische Nukleinsäureliganden oder Aptamere aus einer kombinatorischen Bibliothek von unterschiedlichen Nukleinsäuresequenzen für BrefeldinA-insensitive kleine ARF-GEF-Proteine isoliert werden können. Desweiteren umfasst die Erfindung die hierin beschriebenen Anwendungen der erfindungsgemässen Nukleinsäureliganden, insbesondere die Inhibition der ARF-GEF-Aktivität in vitro oder in Zellen, die Verwendung der Nukleinsäureliganden zur Detektion der kleinen ARF-GEF-Proteine, die Verwendung der Nukleinsäureliganden zur Isolierung ihrer Zielproteine, die Verwendung der Nukleinsäureliganden zum Zweck der Therapie wobei die Applikation der Nukleinsäureliganden durch exogene Zugabe oder durch Expression der Nukleinsäureliganden von geeigneten Vektoren in den Zellen erreicht werden kann. ]

Wie in den Beispielen genauer erläutert werden wird, wurden die erfindungsgemäßen Nukleinsäuren erhalten vermittels der sogenannten in vitro Selektion ausgehend von einer kombinatorischen Bibliothek unterschiedlicher Nukleinsäuresequenzen. Die bei der in vitro Selektion erhaltenen, an ein Zielmolekül bindenden Nukleinsäuren werden generell und auch hierin als Nukleinsäureliganden bezeichnet. Die Techniken zur Isolierung von Nukleinsäuren durch in vitro Selektion sind dem Fachmann bekannt und in der Literatur beschrieben (Methods Enzymol. 267 (1996); Klug und Famulok, Mol. Biol. Rep. 20 (1994), 97-107; Conrad et al., Mol Div. 1 (1995), 69-78; Williams und Bartel, Nucleic Acid Mol. Biol. 10 (1996), Soukup und Breaker, Curr. Opin. Struct. Biol. 10 (2000), 318-325). Bei dem Verfahren zur in vitro Selektion wird dabei grundsätzlich so vorgegangen, dass eine Vielzahl verschiedener Nukleinsäuresequenzen (DNA, RNA und andere) mit einem Zielmolekül (engl. target) in Verbindung gebracht wird und die an das target bindende(n) Nukleinsäure(n) isoliert, wird/werden, wobei bevorzugterweise die nicht-bindenden Nukleinsäuren entfernt werden, und sodann diese Nukleinsäure(n), die gegebenenfalls in ihrer Sequenz geändert wird/werden und damit wieder eine Vielzahl von Nukleinsäuren zur Verfügung gestellt wird, erneut mit dem target in Kontakt gebracht wird. Diese Schritte können mehrfach wiederholt werden.

Nukleinsäuren oder Nukleinsäureliganden im Sinne dieser Erfindung umfassen bevorzugt DNA, RNA oder chemisch modifizierte Formen von DNA oder RNA. Noch bevorzugter gehören die Nukleinsäureliganden zu einer der Klassen der Aptamere, Aptazyme, Ribozyme, Intramere, natürliche Nukleinsäureliganden oder sind natürlich vorkommende Protein-bindende Nukleinsäuren. Besonders bevorzugt sind dabei die Aptazyme, Intramere und die Aptamere, von den dreien wiederum insbesondere die Gruppe der Intramere. Unter natürlichen Nukleinsäuren sollen hierin auch solche Nukleinsäuren verstanden werden, sei es als Ausgangmaterialien (teilweise oder vollständig) für in vitro Selektionsverfahren oder sei es als erfindungsgemäße Nukleinsäuren, die eine Sequenz aufweisen, die im Transkriptom einer Zelle bzw. eines Organismus enthalten ist, wobei unter Transkriptom hierein insbesondere die Gesamtheit der exprimierten Nukleinsäuren verstanden wird. Im Falle der Verwendung von natürlichen Nukleinsäuren ist besonders bevorzugt, wenn statt randomisierten Bibliotheken in Selektionsprozessen natürliche Bibliotheken einzusetzen, wie z.B. cDNA-Fragmente transkribierter RNAs.

Unter vitro Selektion soll hierin insbesondere auch der Prozess verstanden werden durch den die erfindungsgemäßen Nukleinsäureliganden, bevorzugt aus der Gruppe der Aptamere, der Intramere, der Aptazyme oder der Ribozyme, noch bevorzugter aus der Gruppe der Aptamere und Aptazyme, durch in vitro Selektion aus kombinatorischen Bibliotheken von Nukleinsäuren mit unterschiedlichen Sequenzen isoliert werden können.

Unter Aptameren (Ellington uns Szostak, Nature 346 (1990), 818-822) sind im Sinne dieser Erfindung einzelsträngige Nukleinsäureliganden zu verstehen die aus kombinatorischen Bibliotheken von randomisierten Nukleinsäuren.durch in vitro Selektion isoliert werden. Dieser Prozess wird manchmal auch als SELEX ("systematic evolution of ligands by exponential enrichment") bezeichnet (Tuerk und Gold, Science 249 (1990), 505-519). Diese aus ssDNA oder ssRNA bestehenden Aptamere besitzen sehr hohe Affinitäten und Spezifitäten für ihre Antigene. Bis dato wurden Nukleinsäureliganden für kleine, organische Verbindungen, Peptide, Proteine, oder sogar komplexe Strukturen wie Viren und Zellen isoliert (Übersichtsartikel: Gold et al., Annu. Rev. Biochem. 64 (1995), 763-797; Ellington und Conrad, Biotechnol. Annu. Rev. 1 (1995), 185-214; Famulok, Curr. Opin. Struct. Biol. 9 (1999), 324-329). Das hohe Potential der Technologie liegt in dem rein *in vitro* stattfindenden Prozess der Aptamerselektion. Die Nukleinsäureliganden werden aus kombinatorischen Bibliotheken von bis zu 10¹⁵ individuellen Sequenzen durch wiederholte Zyklen aus Kontakt mit dem Antigen, Abtrennung aller nicht bindenden Nukleinsäuren und enzymatischer Amplifikation der mit dem Zielmolekül interagierenden Moleküle angereichert.

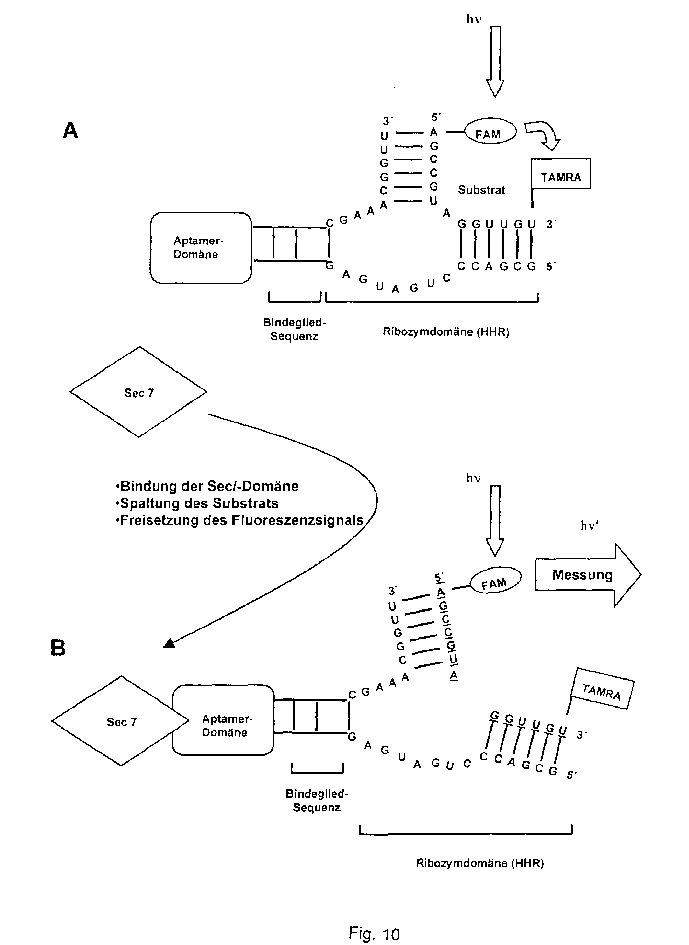

Aptazyme im Sinne der vorliegenden Erfindung, sind Hybridmoleküle aus Ribozymen und Aptameren und bestehen dementsprechend aus einer katalytischen und einer ligandenbindenden Nukleinsäuredomäne. Dabei wird die Aktivität des Ribozyms durch die Bindung des Liganden an die ligandenbindende Nukleinsäuredomäne (allosterisches Zentrum) reguliert, d.h. aktiviert oder inhibiert. Die Aptazymtechnologie ist dem Experten bekannt und beispielsweise beschrieben in Soukup und Breaker, Curr. Opin. Struct. Biol. 10 (2000), 318-325. Dabei können Aptazyme durch rationales Design aus einem Ribozymteil und dem allosterischen Zentrum modular aufgebaut werden oder de novo durch in vitro Selektion isoliert werden. Beispielsweise wurde ein Aptazym aus einem Hammerhead-Ribozym, einem kleinen katalytischen Motiv, das RNA-Sequenzen sequenzspezifisch spalten kann (Forster und Symons, Cell 49 (1987), 211-220; Haseloff und Gerlach, Nature 334 (1988), 585-591), und einer Aptamerdomäne, die spezifisch an ATP bindet, so konstruiert, dass die Bindung von ATP zu einer allosterischen Inhibition der katalytischen Aktivität führte (Tang und Breaker, Chem. Biol. 4 (1997), 453-459, Tang und Breaker, Nucleic Acid Res. 26 (1999), 4222-4229). Andererseits konnten auch Aptazyme durch in vitro Selektion aus kombinatorischen Bibliotheken von unterschiedlichen Nukleinsäuresequenzen isoliert werden, die eine neuartige Bindungsdomäne für einen Liganden enthielten (Robertson und Ellington, Nat. Biotechnol. 17 (1999), 62-66,Koizumi et al., Nat. Struct. Biol. 6 (1999), 1062-1071). Aptazyme im Sinne dieser Erfindung können beispielsweise solche sein, die eine Liganden-Bindungsstelle für kleine ARF-GEF-Proteine enthalten. Derartige Aptazyme können auf der Grundlage der erfindungsgemäßen Nukleinsäuren dadurch hergestellt werden, dass diese mit einem Ribozymteil und dem allosterischen Zentrum modular aufgebaut werden. Eine schematische Darstellung eines Aptazyms ist in Abbildung 10 dargestellt

Unter natürlichen Nukleinsäureliganden sollen hierin in Ergänzung zu dem vorstehend Gesagtem auch solche Nukleinsäuren, Nukleinsäureliganden oder Teile davon, d.h. Nukleinsäuremotive, verstanden werden, die als solche in den Genomen von Organismen kodiert werden und die Fähigkeit besitzen an ein Zielprotein zu binden. Beispiele sind unter vielen anderen die HIV-Tar-RNA, die an das HIV Tat-Protein bindet, oder das HIV-RRE-RNA, die an das HIV-REV-Protein bindet. Dem Fachmann sind eine Fülle solcher natürlicher Nukleinsäureliganden bekannt. Bislang unbekannte Nukleinsäureliganden für bestimmte Zielproteine können auch aus kombinatorischen Bibliotheken von natürlichen Nukleinsäuren unterschiedlicher Sequenz, die beispielsweise die genomische RNA eines Organismus, eines Organs oder einer Zelle repräsentieren, durch in vitro Selektion isoliert werden. Die Herstellung solcher kombinatorischer Bibliotheken ist dem Fachmann bekannt und beispielsweise beschrieben in Singer et al., Nucleic Acid Res. 25 (1997), 781-786. Auf diese Weise konnten beispielsweise genomische DNA-Sequenzen, die den Transkriptionsfaktor TFIIIA von Xenopus laevis binden, isoliert werden (Kinzler und Vogelstein, Nucleic Acid Res. 17 (1989), 3645-3653). In einem anderen Experiment konnten aus mRNA-Fragmenten aus dem Transkriptom des Bakteriums E.coli Liganden für das Bakteriophagen MS2 Protein isoliert werden (Shtatland et al., Nucleic Acids Res. 28 (2000), E93). Dabei wurden überraschenderweise neue, bislang unbekannte Interaktionen beispielsweise mit den mRNAs des rffG-Gens oder des ebgR-Gens gefunden. Dies zeigt, dass neue Nukleinsäure-Liganden für Proteine aus Bibliotheken, die aus dem Transkriptom eines Organismus aufgebaut sind, isoliert werden können.

Unter Intrameren sollen hierin solche Aptamere oder natürliche Nukleinsäuresequenzen oder natürliche Nukleinsäureliganden verstanden werden, die neben der reinen Bindung ihres Liganden spezifisch für den Einsatz im intrazellulären Milieu konstruiert worden sind. Ganz allgemein wird dabei so vorgegangen, dass zu den erfindungsgemäßen Nukleinsäuren weitere Sequenzen hinzugefügt werden, die dazu führen, dass die erfindungsgemäße Nukleinsäure in einem intrazellulären Milieu stabil ist und/oder exprimiert wird. Typischerweise können derartige zusätzliche Nukleinsäuren, die den vorstehend genannten Anforderungen genügen bzw. die entsprechenden erforderlichen Eigenschaften verleihen, bereits dadurch angefügt werden, dass die erfindungsgemäße Nukleinsäure in ein Expressionssystem eingefügt wird. Beispielsweise können durch Verwendung rein viraler Expressionssysteme durch Anfügen von viralen RNA-Sequenzen und der Verwendung von viralen Promotoren eines kombinierten Systems aus dem Vacciniavirus und dem T7-Bakteriophagen Aptamere stabilisiert, mit hoher Transkriptionseffizienz und lokalisiert im Zytoplasma durch virale Polymerasen transkribiert werden und dort ihre Zielmoleküle binden (Blind et al., Proc. Natl. Acad. Sci. USA 96 (1999), 3606-3610). Unter Intrameren sollen hierin insbesondere solche in vitro selektierten Aptamere verstanden werden, die durch Stabilisierung, Lokalisation, Expression, Anfügen natürlicher oder nicht-natürlicher Nukleinsäuresequenzen, chemische Modifikationen oder andere Maßnahmen zum Zwecke der funktionalen Modulation der Aktivität eines intrazellulären Zielmoleküls optimiert und eingesetzt werden. Andere Expressionssysteme, die zur Transkription von Intrameren verwendet werden können, sind beispielsweise eukaryontische RNA-Polymerase-II-abhängige Systeme. So wurden Aptamere gegen einen am Splicing in Drosophila melanogaster beteiligtes Protein (B52) über einen Hitzeschockpromotor induzierbar in transgenen Fliegen exprimiert (Shi H. et al., Proc. Natl. Acad. Sci USA 96 (1999), 10033-10038). Ebenso sind viele Expressionssysteme bekannt, die für die Transkription intrazellulärer Ribozyme benutzt wurden. Beispielsweise wurden RNA-Polymerase-III-Promotoren für die Transkription der RNA-Ribozyme eingesetzt. Zu diesem Zweck wurde die funktionalen Ribozym-Sequenzen meist im Kontext von Teilen kleiner natürlicher RNA-Moleküle in cis eingesetzt. Durch Anfügen von kompakten Sequenzen aus tRNA oder U6-RNA konnten sehr hohe Transkriptionsausbeuten von Ribozymtranskripten erzielt werden (Lee et al., Gene Ther. 2 (1995),377-384, Zouh et al., Thompson et al., Nucleic acid res. 23, 2259-2268; Bertrand et al., RNA 3 (1997), 75-88). Weitere auf Polymerase-III-Promotoren, die sich für die Expression von RNA in Zellen eignen sind dem Fachmann bekannt und inder Literatur beschrieben (Übersichtsartikel: Couture und Stinchcomb, TIG 12 (1996), 510-514; Rossi, Tibtech 13 (1995), 301-305; Bramlage et al., Tibtech 16 (1998), 434-438).. Sie sind für die Transkription vieler kleiner RNA-Moleküle in eukaryontischen Zellen verantwortlich und in allen Zelltypen aktiv. Zudem sind sie in homologen RNA-Transkriptionseinheiten ubiquitär in allen eukaryontischen Spezies zu finden. Pol-III-Promotoren steuern die Transkription kleiner RNA wie tRNA, snRNA, snoRNA, 5S rRNA, oder kleiner viraler RNA, wie der adenoviralen VA-RNA. Ein weiterer Vorteil der RNA-Pol III-Promotoren sind ihre äußerst hohen Transkriptionsaktivitäten. Natürliche Transkripte akkumulieren in Zellen auf bis zu 10⁵ bis 10⁶ Moleküle pro Zelle. Beispiele für RNA-Polymerase III-Promotoren sind die Promotoren von 5S-RNA-Genen (Typ 1) (Specht et al., Nucleic Acid Res. 19 (1991), 2189-2191), tRNA-Promotoren (Typ 2) (Thompson et al., Nucleic Acid Res. 23 (1995), 2259-2268; Sullenger et al., J. Virol. 65 (1991), 6811-6816) U6 snRNA (Typ 3) (Das et al., EMBO J. 7 (1988), 503-512, Lobo und Hernandez, Genes Dev. 58 (1989), 55-67) oder andere nicht unter die Klassifizierung 1 bis 3 fallende Pol-III-Promotoren, wie z.B. 7SL-RNA-Promotoren (Bredow et al., Gene 86 (1989), 217-225). Für die Expression von RNA-Molekülen wurden bisher einige dieser Promotoren, unter anderem tRNA, U6 snRNA- oder VA1-RNA-Promotoren verwendet (Übersichtsartikel: Rossi, Tibtech 13 (1997), 301-305; Bramlage et al., Tibtech 16 (1998), 434-438).

Ein weiteres geeignetes Expressionsystem stellt das sogenannte Pol III - Expressionssystem dar. Das Pol III-Expressionssystem umfasst dabei eine Nukleinsäuresequenz, die in 5'- 3'- Richtung die folgenden Elemente umfasst:
ein C1-Motiv,
eine A1-Box,
ein C2-Motiv,
eine A3-Box, und
einen Terminator,
wobei
das C1-Motiv und das C2-.Motiv zusammen eine Helix ausbilden;
die A1-Box k Basen umfasst, wobei k unabhängig von 1 und m eine ganze Zahl von 0 bis 100 ist;
die A2-Box I Basen umfasst, wobei I unabhängig von k und m eine ganze Zahl von 0 bis 100 ist, und
die A3-Box m Basen umfasst, wobei m unabhängig von k und I eine ganze Zahl von 0 bis 20
ist.

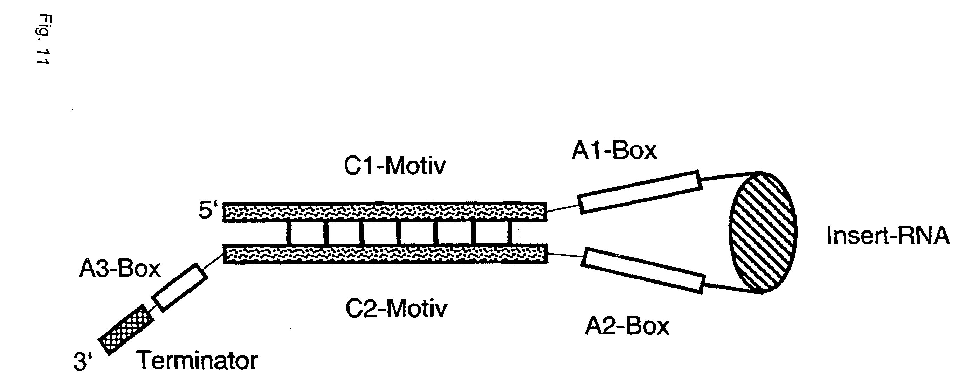

Ein derartiges Pol III-Expressionssystem ist in Fig. 11 dargestellt.

Um die Möglichkeiten der Aptamertechnologie intensiver nutzen zu können, können chemische Derivate der Desoxyribonuklein- und Ribonukleinsäuren entwickelt werden. Beispielsweise können die Nukleinsäuren gegen Degradation durch Nukleasen stabilisiert werden. Eine derartige Stabilisierung kann an verschiedenen Stellen des Nukleinsäuremoleküls erfolgen. So ist eine Modifizierung der Verbindungsgruppe zwischen den Nukleobasen, d.h. der Phosphodiesterbindung, aber auch des Zuckerrestes oder der Basen oder Kombinationen dieser Maßnahmen möglich. Die unterschiedlichen Möglichkeiten chemische veränderte Nukleinsäuren herzustellen sind dem Fachmann bekannt und ausführlich in der Literatur beschrieben (Übersichtsartikel: Uhlmann E. und Peyman A., Chem Reviews 90 (1990), 543-584; Eaton B.E. und Pieken W.A., Annu Rev Biochem 64 (1995), 837-863; Eaton B. E., Curr Opin Chem Biol 1 (1997), 10-16). Insbesondere bevorzugt sind die mit der *in vitro* Transkriptionsreaktion des Selektionsprozesses kompatiblen 2'-Amino- und 2'-Fluoro-Deoxyribonukleotide. In Anwesenheit von Nukleasen erhöhen sich die Halbwertszeiten der modifizierter Moleküle gegenüber natürlichen Nukleinsäuren von Minuten auf mehrere Stunden (Lee S.-W. und Sullenger B.A., Nat Biotechnol 15 (1997), 41-45; Kubik et al., J. Immunol. 159 (1997), 259-267; Pagratis et al., Nat. Biotechnol. 15 (1997), 68-73). Es konnte auch gezeigt werden, dass die nachträgliche Modifikation der Zuckerreste von Purinen mit 2'-O-Methylgruppen und das Anfügen von kurzen Phosphorothioatgruppen an den 5'- und 3'-Enden die Stabilität von RNA-Aptameren in biologischen Medien deutlich erhöhen können (Green et al., Chem. Biol. 2 (1995), 683-695). Die Ribonukleinsäureliganden erreichen Halbwertszeiten von bis zu 72 Stunden.

Durch Einführung von chemischen Verbindungsgruppen können Nukleinsäureliganden strukturell stabilisiert werden. Ein Beispiel ist die strukturelle Stabilisierung von zwei kurzen Oligonukleotiden aus dem minimalen RBE (Rev binding element) durch eine verbindende Stilbendicarboxyamid-Brücke (Nelson et al, Biochemistry 35 (1996), 5339-5344). Das RBE in der genomischen RNA des HIV-Virus bindet an das virale Rev-Protein. Rev ist daraufhin verantwortlich für den Transport der viralen RNA aus dem Zellkern in das Zytoplasma. Ohne die chemische Verknüpfung waren die beiden Oligonukleotide nicht in der Lage thermisch stabile Strukturen über Basenpaarung auszubilden. Mit der Stilbendicarboxyamid-Brücke war die Struktur stabilisiert und band das Rev-Protein mit gleichen Affinitäten wie das virale RBE. Diese Methode ist insbesondere nützlich zur Herstellung von stark verkürzten funktionalen Nukleinsäureliganden.

Die erfindungsgemäßen Nukleinsäuren sind grundsätzlich und auch bei den verschiedenen möglichen und insbesondere den hierin offenbarten Verwendungen oder Anwendungen nicht auf die spezifischen offenbarten Sequenzen beschränkt. Vielmehr ist es im Rahmen der Fähigkeiten der Fachleute auf diesem Gebiet, ausgehend von den konkret offenbarten Nukleinsäuren, diese zu modifizieren. Derartige Nukleinsäuren werden hierin auch als modifizierte Nukleinsäuren bezeichnet. Wann immer hierin oder in den Ansprüchen von den erfindungsgemäßen Nukleinsäuren und deren Anwendung oder Verwendung die Rede ist, beinhaltet dies auch gleichzeitig immer die modifizierten Nukleinsäuren und deren Anwendung oder Verwendung. Im Rahmen einer derartigen Modifizierung können Nukleobasen chemisch modifiziert werden oder auch einzelne oder mehrere der Nukleobasen ausgetauscht oder deletiert werden. Die Herstellung von Nukleinsäuren, bei denen Nukleobasen ausgetauscht oder deletiert werden und trotzdem die gleichen Bindungseigenschaften wie die ursprünglichen Nukleinsäureligenden aufweisen sind dem Fachmann bekannt und in der Literatur beschrieben. Ein Beispiel ist die Erzeugung von mutierten und verkürzten Derivaten eines Aptamers gegen den Transkriptionsfaktor NFκB (Lebruska und Maher, Biochemistry 38 (1999), 3168-3174). Um Mutanten eines Aptamers (Aptamer 3) zu isolieren, die wie das ursprüngliche Aptamer 3 an den Transkriptionsfaktor NFkB binden, wurde eine Bibliothek von Mutanten hergestellt, die an jeder Position die korrekte Base mit einer Wahrscheinlichkeit von 0,7 enthielt und die übrigen drei Basen mit einer Wahrscheinlichkeit von je 0,1. Durch in vitro Selektion wurden Mutanten isoliert, die trotz Austausch von Nukleobasen immer noch an den Transkriptionsfaktor banden. Um Deletionen zu erzeugen wurde das ursprüngliche Aptamer durch alkalische Hydrolyse partiell gespalten und in Bindungsstudien solche Fragmente isoliert, die trotz der Deletionen noch an den Transkriptionsfaktor NFκB banden. Auf diese Weise konnten verkürzte Formen des ursprünglichen Aptamers hergestellt werden. Es können aber auch zu den erfindungsgemäßen Sequenzen, wie bereits im Zusammenhang mit den Aptazymen und Intrameren ausgeführt, weitere Nukleotide hinzugefügt werden. Insoweit stellen die konkreten Sequenzen der hierin offenbarten Nukleinsäuren einen Rahmen dar, von dem ausgehend weitere Sequenzen denkbar sind und der nur dadurch begrenzt wird, dass auch die solchermaßen modifizierte Nukleinsäure nach wie vor an den Guaninnukleotid-Austauschfaktor bindet und bei bevorzugten Ausführungsformen auch die GDP/GTP-Austauschfunktion moduliert, insbesondere inhibiert.

Bei dem Guaninnukleotid-Austauschfaktor kann es sich grundsätzlich und auch bei den verschiedenen möglichen und insbesondere den hierin offenbarten Verwendungen oder Anwendungen um einen solchen handeln, der zur Gruppe der Guaninnukleotid-Austauschfaktoren für ADP-Ribosylierunsfaktoren (ARF) mit einem Molekulargewicht von etwa 50 kDa oder weniger gehören. Die ADP-Ribosylierungsfaktoren gehören zu einer Familie von regulatorischen GTPasen, die auch als G-Proteine bezeichnet werden. Bei dem Guaninnukleotid-Austauschfaktor kann es sich auch um einen solchen handeln, der nicht durch Brefeldin A inhibierbar ist. Weiterhin kann es sich bei dem Guaninnukleotid-Austauschfaktor um einen solchen handeln, der ausgewählt ist aus der Gruppe, die Cytohesin-1, Cytohesin-2 (ARNO), Cytohesin-3 (GRP-1, ARNO3), Cytohesin-4 oder EFA6 umfaßt,
wobei Cytohesin-1 und Cytohesin-2 ganz besonders bevorzugt sind. Schließlich kann der Guaninnukleotid-Austauschfaktor auch ein solcher sein, der eine Sec7-Domäne oder ein Homolog dazu aufweist. Schließlich kann es sich bei dem Guaninnukleotid-Austauschfaktor um einen solchen handeln, der eine beliebige Kombination aus zwei oder mehreren der vorstehenden Merkmale umfasst.

Die Erfindung betrifft in einem weiteren Aspekt ein Expressionssystem, das mindestens eine oder mehrere der erfindungsgemäßen Nukleinsäuren oder Modifikationen davon umfasst. Unter Expressionssystem sollen hierin insbesondere Vektoren verstanden werden, die geeignete Expressionskassetten für die erfindungsgemäßen Nukleinsäuren enthalten und die Einschleusung und/oder die Expression der erfindungsgemäßen Nukleinsäuren in Zellen ermöglichen. Besonders bevorzugt sind hier eukaryontische Zellen. Geeignete Vektoren zur Einschleusung der Gene sind dem Fachmann bekannt. Sie können bloße DNA-Vektoren wie Plasmide, Cosmide, BACs ("bacterial artificial chromosomes"), YACs ("yeast artificial chromosomes"), MACs ("mammalian artificial chromosomes") umfassen. Desweiteren sind beispielsweise virale Vektoren, wie Sindbisviren, Vacciniaviren, Adenoviren, AAV oder Retroviren möglich. Desweiteren betrifft die Erfindung Expressionskassetten, die zumindest eine der erfindungsgemäßen Nukleinsäuren bzw. die Gene für die erfindungsgemäßen Nukleinsäureliganden, bevorzugt RNA-Liganden, umfassen, wie die in Beispiel 4 beschriebene (vgl. auch Blind et al., Proc Natl. Acad. Sci. USA 96 (2000), 3606-3610; PCT/EP/00/02727). Andere Beispiele für Expressionskassetten sind beschrieben in Good et al., Gene Ther. 4 (1997), 45-54 oder Bertrand et al., RNA 3 (1997), 75-88. Dabei umfassen derartige Expressionskassetten typischerweise geeignete Promotoren zur Expression der Nukleinsäureliganden und können zusätzlich zu den Nukleinsäureliganden noch für Sequenzen kodieren, die dem Hybridmolekül aus Nukleinsäureligand und zusätzlichen Sequenzen intrazelluläre Stabilität verleihen oder seine intrazelluläre Lokalisierung steuern (Blind und Famulok, DE 100 46 913.2).

Die erfindungsgemäßen Nukleinsäuren können sowohl als solches als auch in Expressionssystemen oder Expressionskassetten verwendet werden. Eine besonders wichtige Anwendung ist der Bereich der Medizin, wobei sowohl therapeutische als auch diagnostische Anwendungen möglich sind, die auf den hierin beschriebenen Wechselwirkungen und Mechanismen beruhen. In so weit können die erfindungsgemäßen Nukleinsäuren einzeln oder in beliebigen Kombinationen zur Herstellung eines Medikamentes verwendet werden. Ganz besonders bevorzugt ist dabei die Verwendung zur Herstellung eines Medikamentes zur Prophylaxe und/oder Therapie von Krankheiten oder Krankheitsbildern, bei denen das Medikament die durch β-2-Integrine vermittelte Adhäsion von Immunzellen beeinflusst. Bei den Immunzellen handelt es sich bevorzugt um solche Zellen, die ausgewählt sind aus der Gruppe, die Leukozyten, Phagozyten, T-Lymphozyten, myeloide Zellen und NK-("natural killer") Zellen umfasst.

Die Verwendung der erfindungsgemäßen Nukleinsäuren kann bei der Therapie sowohl in der direkten Verabreichung derselben erfolgen als auch im Rahmen der Gentherapie. Im Falle der Gentherapie werden dabei die die erfindungsgemäßen Nukleinsäuren enthaltenden Vektoren, Expressionssystem und Expressionskassetten von besonderer Bedeutung sein. Bei der gentherapeutischen Verwendung der erfindungsgemäßen Nukleinsäuren ist dabei der grundsätzliche Anwendungsaspekt der, dass eine die erfindungsgemäßen Nukleinsäuren enthaltende Expressionskassette durch einen geeigneten Vektor in Zellen eines eukaryontischen Organismus, insbesondere in humane Zellen, eingeschleust werden. Von der eingeschleusten Expressionskassette können die erfindungsgemäßen Nukleinsäuren dann in den Zellen exprimiert werden und dort die Funktion der ARF-GEF-Proteine inhibieren. Als Vektoren für gentherapeutische Ansätze können freie DNA, in Trägermaterialien wie kationische Liposomen verpackte DNA, die weiter unten näher beschrieben sind, oder virale Vektoren wie Adenoviren, adeno-assoziierte Viren, Retroviren, Herpesviren, Polyomaviren, Papillomaviren oder Vacciniaviren verwendet werden (Übersichtsartikel: Walther und Stein, Drugs 60 (2000), 249-271; Buchschacher und Wong-Staal, Blood 95 (2000), 2499-2504; Krauzewicz und Griffin, Adv. Exp. Med. Biol. 465 (2000), 73-82; Virtanen et al., Adv. Exp. Med. Biol. 465 (2000), 423-429). Geeignete Vektoren für die erfindungsgemäße Nukleinsäuresequenz sind den Fachleuten bekannt. Als freie DNA-Vektoren können unter anderem Plasmide und Cosmide verwendet werden, die durch Elektroporation, Lipofektion oder CaPO₄-Präzipitation in die Zellen eingeschleust werden (Übersichtsartikel: Gregoriadis, Pharm. Res. 15 1998, 661-670). Eine Erweiterung dieser Methode ist die Verwendung episomal replizierender Plasmide, die den "origin of replication" des Ebstein-Barr Virus (OriP) tragen und das EBNA-1-Antigen exprimieren. Diese Vektoren replizieren extrachromosomal in Primaten- und Hund-Zelllinien und können dort dauerhaft persistieren (Yates et al., Nature 313 (1985), 812-815; Chittenden et al., J. Virol. 63 (1989), 3016-3025).Eine andere Methode, fremdes genetisches Material in eukaryontischen Zellen dauerhaft zu replizieren, ist die Verwendung von sogenannten Minichromosomen. Diese großen DNA-Moleküle tragen wie natürliche Chromosomen Zentromer- und Telomersequenzen und werden in der Mitose dupliziert und an die Tochterzellen weitergegeben. Ihre Größe (im Megabasenbereich) erlaubt zudem die Klonierung sehr großer DNA-Fragmente von mehreren 100 000 Basen. Minichromosomen wurden für Bakterien-, Hefe- und Säugerzellen entwickelt (YACs und BACs, siehe: Grimes und Cooke, Hum. Mol. Genet. 7 (1998), 1635-1640; Amemiya et al., Methods Cell. Biol. 60 (1999),:235-258; Brown et al., Trends Biotechnol. 18 (2000), 218-223). Die hier aufgeführten Vektoren sind nicht limitierende Beispiele für Vektorsysteme, die in der Literatur beschrieben und den Fachleuten bekannt sind.

Häufig benutzte virale Vektoren sind Retroviren, deren RNA-Genom nach der reversen Transcription als DNA stabil in das Genom des Wirts integriert. Am häufigsten werden auf MoMuLV basierende Vektoren eingesetzt, die allerdings nur proliferierende Zellen infizieren können. Daher wurden auch auf Lentiviren (u.a. HIV) basierende Systeme entwickelt, mit denen auch sich nicht-teilende Zellen infiziert werden können (Übersichtsartikel: Miller, Hum Gene Ther. 1 (1990), 5-14; Gordon und Anderson, Curr. Opin. Biotechnol. 5 (1994), 611-616). Eine andere häufig verwendete Klasse sind adeno-assoziierte Viren (AAV). Diese ssDNA-Viren zeichnen sich unter anderem durch den Vorteil aus, dass sie genetisches Material an einer definierten Stelle im Chromosom 19 integrieren können (Übersichtsartikel: Grimm und Kleinschmidt, Hum. Gene. Ther. 10 (1999), 2445-2450).

Die direkte Verabreichung, gegebenenfalls aber auch die Verabreichung im Rahmen der Gentherapie, der erfindungsgemäßen Nukleinsäuren kann dabei vermittels aller galenischer Techniken und Zusammensetzungen, die eine oder mehrere der erfindungsgemäßen Nukleinsäuren in ihren verschiedenen Ausführungsformen umfassen, erfolgen. Beispielhaft sei hier auf Lösungen, Pulver, Tabletten, Gele und dergleichen verwiesen. Dabei wird typischerweise die erfindungsgemäße Nukleinsäure zusammen mit einem Stabilisator, Adjuvanz, pharmazeutisch akzeptablen Träger, Konservierungsmittel, Verdünnungsmittel, Träger oder einer Kombination davon hergestellt und/oder verabreicht. Methoden zur Einschleusung der erfindungsgemäßen Nukleinsäuren in Zellen sind dem Fachmann bekannt. Die erfindungsgemäßen Nukleinsäuren können direkt beispielsweise als liposomale Formulierungen verwendet werden. Transfectionsprotokolle mit kationischen Liposomen sind beschrieben in Ausubel et al. (Current Protocolls in Molecular Biology (1991), Wiley Interscience , New York) oder Sambrook et al. (Molecular cloning - a laboratory manual, 2^{nd} Ed. (1989), Cold Sprin Harbor Laboratory Press, Cold Spring Harbor). Detaillierte Beschreibungen über die Art von kationischen Amphiphilen (liposomen), die sich auch für die in vivo Verabreichung von therapeutischen Nukleinsäuren eignen sind beschrieben in (Felgner et al. (Proc Natl Acad Sci USA (1987), 7413-7417), Behr et al., Proc. Natl. Acad Sci USA 86 (1994), Epand et al., (US 5,283,185) oder Lee et al. (US 5,925,628). Andere Transfektionsreagention für Nukleinsäuren sind beispielsweise Dendrimere (Tang et al., Bioconjugate Chem 7 (1996), 703) oder andere polykationische Polymere (z.B. beschrieben in Illum, US 5,744,166). Eine weitere Möglichkeit zur Einführung von Nukleinsäuren in Zellen sind Hybridmoleküle aus der Nukleinsäure und kurzen Signalpeptiden, die natürlicherweise z.B. in der basischen Domäne des HIV Tat-Proteins oder der Helix 3 des Antennapediaproteins (Litstelle) vorkommen. Diese Signalpeptide können Makromoleküle über einen bislang noch nicht im Detail verstandenen Prozeß direkt durch die Zytoplasmamembran in das intrazelluläre Kompartiment einschleusen. Die Verendung solcher Signalpeptide wurde beispielsweise beschrieben von Troy und Shelanski (US 5929042) oder von Lin. und Hawiger. (US 5,807,746). Die angeführten Reagenzien dienen der Beschreibung der vielfältigen Möglichkeiten zur Einschleusung von Nukleinsäuren in Zellen, die dem Fachmann bekannt sind, und sollen hier als nicht limitierende Beispiele verstanden werden.

Mit den erfindungsgemäßen Nukleinsäuren wird erstmals ein Inhibitor für die Guaninnukleotid-Austauschfaktoren der ARF-Proteine, wie sie hierin beschrieben sind, bereitgestellt. Die Verwendungen dieses Inhibitors entsprechen denjenigen der erfindungsgemäßen Nukleinsäuren. Gleiches gilt für die pharmazeutische Ausgestaltung einer den Inhibitor enthaltenden insbesondere pharmazeutischen Zusammensetzung oder Mittel, das neben dem Inhibitor noch weitere pharmazeutisch wirksame Verbindungen enthalten kann. Des weiteren kann ein derartiges Mittel noch übliche galenische Hilfsstoffe enthalten, die z.B. ausgewählt sind aus der Gruppe, die Puffer, Konservierungsstoffe, Chelatoren, Stabilisierungsmittel, Maskierungsmittel, Füllstoffe, Sprengmittel, pharmazeutisch akzeptable Träger und Isotonizitäts-anpassende Verbindungen umfaßt. Mit dem erfindungsgemäßen Inhibitor steht darüber hinaus auch ein Werkzeug zur Verfügung, um das intrazelluläre Geschehen im Zusammenhang mit der Wechselwirkung von ARF-GEF's allgemein darzustellen und zu untersuchen, wobei infolge der Größe des Inhibitors und seiner chemischen bzw. molekularen Natur gegenüber der Verwendung von geänderten Wechselwirkungspartnern wie mutierten oder überexprimierten Wechselwirkungspartnern von diesem ausgehende Wechselwirkungen auf andere Systeme innerhalb der Zelle ausgeschlossen werden können.

Eine weitere Anwendung der erfindungsgemäßen Nukleinsäuren liegt im diagnostischen Bereich. Nukleinsäureliganden können wie Antikörper in einer Vielzahl von diagnostischen Applikationen weingesetzt werden (Übersichtsartikel: Hesselberth et al., J Biotechnol 74 (2000), 15-25; Brody und Gold, Biotechnol 74 (2000), 5-13; Jayasena Clin Chem 45 (1999), 1628-1650; Osborne et al., Curr Opin Chem Biol 1 (1997), 5-9). Durch die Bindung der erfindungsgemäßen Nukleinsäuren an die Zielstruktur, d.h. den Guaninnukleotid-Austauschfaktor kann dieser markiert werden. Trägt die erfindungsgemäße Nukleinsäure ihrerseits ein Markierung, kann diese und damit der Komplex aus Guaninnukleotid-Austauschfaktor und erfindungsgemäßer Nukleinsäure nachgewiesen werden. Geeignete Markierungen sind den Fachleuten auf dem Gebiet bekannt und umfassen, unter anderem, jene, die aus der Gruppe ausgewählt sind, die radioaktive Markierungen, Markierungen mit Fluoreszenzfarbstoffen, Markierung mit Gruppen wie Biotin oder Digoxygenin oder Markierungen mit Enzymen wie β-Galaktosidase umfasst. Geeignete Fluoreszenzfarbstoffe für die Markierung von Nukleinsäuren sind den Fachleuten bekannt und umfassen u.a. AMCA-X, Fluorescein, Rhodamin, Cy3, Cy 5, Cy 5.5, HEX, D-AMCA, Tetramethylrhodamin oder Texas Red. Der Nachweis des besagten Komplexes mit andern Nukleinsäure/Proteinkomplexen kann beispielsweise durch FACS (Ringquist und Parma , Anal Chem. 70 (1998), 3419-3425), fiberoptische Microarray Sensoren (Lee und Walt, Anal Biochem 282 (2000), 142-146), Kapillarelektrophorese (German I., Anal Chem 70 (1998), 4540-4545) oder Fluoreszenzmikroskopie erfolgen.

Alternativ können die erfindungsgemäßen Nukleinsäuren, der Guaninnukleotidaustauschfaktor oder beide mit Fluoreszenzfarbstoffen markiert sind und die der Wechselwirkung zwischen der Nukleinsäure und dem Guaninnukleotidaustauschfaktor zu einer meßbaren Veränderung des Fluoreszenzsignals führt. Beispielsweise konnte die Bindung von Thrombin an mit Fluoreszenzfarbstoffen markierten anti-Thrombin-Aptameren durch Messung der Änderung der Fluoreszenz-Anisotropy nachgewiesen werden (Potyrailo R. A. et al., Anal Chem 70 (1998), 3419-25). Dabei konnten bis zu 0,7 amol Thrombin nachgewiesen werden. In einer anderen Ausführungsform ist ein Interaktionspartner, die Nukleinsäure oder der Guaninnukleotidaustauschfaktor mit einer fluoreszenzlöschenden Gruppe (Quencher), der andere mit einer fluorophoren Gruppe (Donor) markiert ist, wobei die Wechselwirkung zwischen beiden Interaktionspartnern zur Löschung der Fluoreszenzemission des Donors durch den Quencher führt. Findet die Wechselwirkung statt kann dies durch eine Verminderung des Fluoreszenzsignals gemessen werden. Diese auch als FRET ("fluoreszenz resonanz energy transfer") bezeichnete Methode ist dem Fachmann bekannt und wird auch zur Detektion von Protein/Proteinwechselwirkungen angewendet (Sorkin A. et al., Curr Biol. 10 (2000), 1395-1388; Latif R. und Graves P., Thyroid. 10 (2000), 407-412, 14: Buranda T. et al., Cytometry 37 (1999), 21-31), Dem Fachmann sind die Prinzipien des Nachweises von Interaktionen zwischen Molekülen durch Fluoreszenzmarkierung und die verschiedenen Gestaltungsmöglichkeiten der Nachweise über einfache oder mehrfache Flureszenzmarkierung eines oder beider Interaktionspartner vertraut.

Ebenso kann die Detektion der Komplexe aus den erfindungsgemäßen Nukleinsäuren und dem Guaninnukleotidaustauschfaktor auf geeigneten Sensoren erfolgen, die mit einem der beiden Bindungspartnern beschichtet sind. Die Detektion kann hier über die Zunahme der Masse nach der Bindung des zweiten Bindungspartners erfolgen. Beispielsweise wurden Komplexe aus RNA-Aptameren und dem Enzym 2'-5'-Oligoadenylatzyklase oder RNA-Aptameren und der NS3-Protease des Hepatitis C Virus durch sogenannte SRP-("Surface Plasmon Resonance")-Analysen detektiert (Hartmann et al., J Biol Chem 273 (1998), 3236-3246; Hwang et al., Biochem Biophys Res Commun 279 (2000), 557-562). Andere geeignete Analysemethoden, wie Quarzkristall-Mikrowaagen ("quartz crystal microbalance", QCM) (Kosslinger et al., Biosens Bioelectron 7 (1992), 397-404; Hengerer et al., Biosens Bioelectron 14 (1999), 139-144), sind dem Fachmann bekannt.

In einer besonderen Ausführung kann die Interaktion zwischen dem Protein und dem Nukleinsäureliganden, insbesondere einem Aptazym, durch ein katalytisches Ereignis nachgewiesen werden. Dabei ist die das ARF-GEF-Protein erkennende Nukleinsäuredomäne mit einer Ribozymdomäne in einer Weise verknüpft, dass die Bindung des ARF-GEF-Proteins die Aktivität der Ribozymdomäne allosterisch beeinflusst, d.h. aktiviert oder inhibiert. Die Konstruktion von signalgebenden Aptazymen ist dem Fachmann bekannt und in der Literatur beschrieben (Hesselberth et al., J Biotechnol 74 (2000), 15-25; Marshal und Ellington, Nat Struct Biol 6 (1999), 992-994; Soukup und Breaker, Trends Biotechnol 17 (1999), 469-476). Beispiele für Ribozymdomänen, die in Aptazymkonstrukten eingesetzt worden sind, sind effektoraktivierte Ribozymligasen (Robertson und Ellington Nucleic Acids Res 28 (2000),:1751-1759) oder Hammerheadribozyme (Soukup et al., J Mol Biol 298 (2000), 623-632). Der Nachweis der aktiven Ribozymdomäne kann im Fall der Ribozymligasen, die sich selbst mit einem Oligonukleotid verknüpfen über reverse Transcription PCR mit einem zu dem verknüpften Oligonukleotid komplementären Primer erfolgen (Robertson und Ellington, Nat Biotechnol 17 (1999), 62-66). Aktive Hammerheaddomänen können durch Spaltung eines Substratoligonukleotids beispielsweise in einem FRET-Assay nachgewiesen werden (Jenne et al., Angew. Chem. 111 (1999), 1383-1386). Dabei ist das zu spaltende Oligonukleotid mit einer fluoreszenzgebenden (Donor) und einer fluoreszenzlöschenden Gruppe (Quencher) markiert. Im ungespeltenen Zustand sitzen Donor und Quencher in unmittelbarer Nachbarschaft und das vom Donor emitierte Licht wird vom Quencher absorbiert. Nach der Spaltung wird der Donor freigesetzt und die jetzt von ihm frei emittierte Fluoreszenz kann detektiert werden.

Ebenso können die erfindungsgemäßen Nukleinsäureliganden wie Antikörper in dem Fachmann bekannten ELISA-Applikationen eingesetzt werden. Ein Beispiel für die Anwendung eines Aptamers in einer Elisaapplikation ist der spezifische Nachweis des Proteins VEGF mittels anti-VEGF-Aptameren (Drolet et al., Nat Biotechnol 14 (1996), 1021-1024; Kato et al., Analyst 125 (2000), 1371-1373). Die erfindungsgemäßen Nukleinsäuren können auch zur Detektion der ARF-GEF-Proteine auf Chips als Trägermaterialien eingesetzt werden. Die Anwendung von Nukleinsäureliganden in derartigen Formaten wurde beispielsweise beschrieben in Brody et al. (Mol Diagn 4 (1999), 381-388). Als Probenmaterialien eignen sich dabei unter anderem Körperflüssigkeiten wie Blut, Urin, Lymphflüssigkeit, Vaginalflüssigkeit, Cerebrospinalflüssigkeit, Punktate, Stuhl, Biopsien, zelluläre Proben und Aufschlüsse davon. Daraus ergibt sich der spezifische Nachweis von ARF-GEF-Proteinen in den zu untersuchenden Proben

Die Verwendung der erfindungsgemäßen Nukleinsäuren zu diesen Zwecken kann auch mittels sogenannter Kits erfolgen. Im vorliegenden Fall umfasst ein derartiger Kit zumindest eine der erfindungsgemäßen Nukleinsäuren, die optional bereits mit einer der oben beschriebenen Markierungen versehen sind. Weitere Ausführungsformen umfassen weitere Reagenzien, wie z.B. Positiv- und Negativkontrollen. Negativkontrollen können dabei so ausgebildet sein, dass eine Nukleinsäure im Kit enthalten ist, die an Guaninnukleotid-Austauschfaktoren, oder bestimmten Formen davon, wie sie hierin auch beschrieben sind, nicht binden oder deren Funktion, insbesondere die GDT/GTP-Austausch-Funktion, nicht modulieren, und insbesondere diese nicht inhibieren. Als Positivkontrolle kann in einer weiteren Ausführungsform ein Guaninnukleotid-Austauschfaktor beigefügt werden, an den die in dem Kit ebenfalls vorhandene erfindungsgemäße Nukleinsäure bindet. Sowohl die erfindungsgemäße Nukleinsäure als auch die Guaninnukleotid-Austauschfaktor kann in einer Ausführungsform des Kits dabei entweder einzeln oder gemeinsam in einer Zelle, bevorzugterweise exprimiert oder in einer exprimierbaren Form vorliegen. In weiteren Ausführungsformen kann der Kit weiterhin umfassen Puffer, Waschlösungen und dergleichen.

Die Verwendung der erfindungsgemäßen Nukleinsäuren, um einen Komplex mit den hierin beschriebenen Guaninnukleotid-Austauschfaktoren für ARF-Proteine auszubilden, kann neben dem therapeutischen und diagnostischen Bereich auch im technischen oder präparativen Bereich erfolgen. Dabei können die erfindungsgemäßen Nukleinsäuren an Trägermaterialien wie z.B. Sepharosen, Agarosen, oder magnetische Partikel gekoppelt werden. Diese Affinitätsträgermaterialien können daraufhin zur präparativen Aufreinigung der ARF-GEF-Proteine verwendet werden. Entsprechende Protokolle wie Affinitätspräzibitationen, analog zu Immunpräzipitationen, oder Affinitätschromatographien sind dem Fachmann bekannt. Protokolle für analoge Immunpräzipitationen sind beispielsweise beschrieben in Ausubel et al. (Current Protocolls in Molecular Biology (1991), Wiley Interscience , New York) oder Sambrook et al. (Molecular cloning - a laboratory manual, 2^{nd} Ed. (1989), Cold Sprin Harbor Laboratory Press, Cold Spring Harbor). Nach Auftragen der Probe und Bindung des Wechselwirkungspartners kann das Material mit geeigneten Waschlösungen von unspezifischen Bindungspartnern befreit werden. Anschließend wird der spezifisch gebundene Wechselwirkungspartner, d.h. der Guaninnukleotid-Austauschfaktor, eluiert und optional quantifiziert. Als Elutionsmittel bzw. Elutionsbedingungen werden dabei typischerweise hohe Salzkonzentrationen, Detergenzien wie SDS, chaotrope Agenzien wie Harnstoff oder Guanadiniumhydrochlorid verwendet. Desweiteren kann as Protein durch Hitzedenaturierung der Nukleinsäureliganden, typischerweise zwisch 60°C und 95°C oder Denaturierung der Strkutur der Nukleinsäureliganden durch Komplexierung zweiwertiger lonen beispielsweise durch EDTA eluiert werden. Dem Fachmann sind Protokolle, die zur Auflösung der Interaktion zwischen den Nukleinsäureliganden und dem aufzureinigenden Zielprotein verwendet werden können, bekannt. Als Probe können die vorstehend genannten Proben verwendet werden. Zusätzlich kann infolge des präparativen Charakters dieser Ausführungsform die Probe auch eine Kultivierungsmedium für Zellen sein, die den jeweiligen Bindungspartner bilden, mit oder ohne Zellen (im Falle des Exports des Bindungspartners), bzw. ein Aufschluß von Zellen sein. Ein Protokoll für die Aufreinigung von in CHO-Zellen exprimiertem humanem L-Selektin mittels eines spezifischen Aptamers über Affinitätschromatographie wurde von Romig und Mitarbeitern beschrieben (Romig, J Chromatogr B Biomed Sci Appl 731 (1999), 275-284)

Eine weitere Form der Anwendung der Komplexbildung zwischen einer der erfindungsgemäßen Nukleinsäuren und einem Guaninnukleotid-Austauschfaktor erfolgt in den erfindungsgemäßen Screeningverfahren, die im folgenden beschrieben werden sollten.

Die Erfindung umfasst in einem Aspekt auch die Verwendung der erfindungsgemäßen Nukleinsäuren in Screeningsystemen. Beispielsweise können Bibliotheken von Substanzen, bevorzugt niedermolekularen Substanzen, auf solche Substanzen durchsucht werden, die durch Bindung an einen der beiden Interaktionspartner, d.h. Brefeldin A resistente ARF-GEF-Proteine oder zumindest eine der erfindungsgemäßen Nukleinsäuren, die Interaktion der beiden Interaktionspartner blockieren. Analoge Screeningsysteme wurden beispielsweise zum Screening von Inhibitoren von Protein/Proteinwechselwirkungen entwickelt. So konnten niedermolekulare Inhibitoren für die Wechselwirkung zwischen Cyanovirin-N und dem HIV-Protein gp120 (McMahon et al., J. Biomol. Screen 5 (2000), 169-176) oder niedermolekulare Inhibitoren für die Wechselwirkung zwischen Peptiden einer Phagenbibliothek der Tyrosyl-tRNA Synthase von H. influenza (Hyde-DeRuyscher et al., Chem. Biol. 7 (2000), 17-25) identifiziert werden. Moleküle, d.h. genauer gesagt Inhibitoren, die die erfindungsgemäßen Nukleinsäuresequenzen von den kleinen ARF-GEF-Proteinen verdrängen, könnten funktional äquivalent zu den erfindungsgemäßen Nukleinsäuren sein. Neben der Verwendung der erfindungsgemäßen Nukleinsäuren als Inhibitoren können diese somit auch zur Identifizierung von Verbindungen aus einer Bibliothek dienen bzw. den Mitgliedern dieser Bibliothek eine bestimmte Funktion oder Verwendung zuweisen, nämlich die dass sie eine zu den erfindungsgemäßen Nukleinsäuren funktionsanaloge Wirkung aufweisen und die Wechselwirkung zwischen Guaninnukleotid-Austauschfaktor und ADP-Ribosylierungsfaktor modulieren, genauer inhibieren. Neben dieser Verwendung können die erfindungsgemäßen Nukleinsäuren ebenso wie die in dem erfindungsgemäßen Screening-Verfahren identifizierten Mitglieder der Bibliothek aber auch wertvolle Leitstrukturen sein und somit zur Entwicklung neuartiger Therapeutika dienen. In diesen Screeningsystemen kann die Interaktion zwischen den erfindungsgemäßen Nukleinsäuren und den ARF-GEF-Proteinen wie schon beschrieben über fluoreszenzbasierende Methoden (z.B. FRET) oder über Aptazymkonstrukte nachgewiesen werden. Bei Verdrängung der erfindungsgemäßen Nukleinsäuren durch ein in der Bibliothek vorhandene Substanz wird ein Signal generiert. Die so identifizierte Substanz bindet an das gleiche Interaktionszentrum wie die erfindungsgemäßen Nukleinsäuren, hier die katalytische Domäne des ARF-GEF_Proteins. Dadurch ist die identifizierte Substenz in bezug auf die Bindung ein funktionelles Analog der erfindungsgemäßen Nukleinsäure und mit erhöhter Wahrscheinlichkeit eine Substanz die durch Bindung an das aktive Zentrum auch die Aktivität des ARF-GEF-Proteins inhibieren kann. Ein Vorteil dieses Screeningsystems ist, dass i) weder die Substanzbibliothek noch das Zielprotein markiert werden müssen. Dadurch kann eine erhebliche Rationalisierung des Prozesses erreicht werden. Zudem werden die Eigenschaften der in der Bibliothek enthaltenen Substanzen und des Proteins durch die bei konventionellen Verfahren meist nachträglich eingeführten Markierungen nicht verändert, ii) ist das Screening fokussiert auf das aktive Zentrum, da alle an andere Bereiche des Proteins bindenden Substanzen nicht detektiert werden. Dies erhöht die Chancen auf die Identifizierung von Substanzen mit der gewünschten inhibitorischen Wirkung.

Im folgenden wird die Erfindung anhand der Figuren und Beispiele erläutert, aus denen sich weitere Merkmale, Ausführungsformen und Vorteile der verschiedenen Aspekte der Erfindung ergeben. Dabei zeigt.
- Fig. 1: eine schematische Darstellung des Aktivierungs-/Inaktivierungszyklus der ARF-Proteine,
- Fig. 2: eine vergleichende Übersicht über die Familie der ARF-GEF-Proteine,
- Fig. 3: die Sequenzen der in dem in vitro Selektionsexperiment gegen Cytohesin-1 gefundenen RNA-Aptamere,
- Fig. 4: einen Vergleich der Aminosäuresequenz der Sec-7-Domänen von Gea2, Cytohesin-1 und Cytohesin-2,
- Fig. 5: die Inhibition der GDP/GTP-Austauschaktivität durch eine der erfindungsgemäßen Nukleinsäuresequenzen (Aptamer M69),
- Fig. 6: das TR-Expressionssystem zur Expression von Intrameren (intrazellulären Aptameren),
- Fig. 7: die Inhibition der Adhäsion von Jurkat E6-Zellen durch ihre LFA-1-Integrine an ICAM-1, wenn das Aptamer M69 durch das TR-Expressionssystem in den Zellen exprimiert wird,
- Fig. 8: Fluoreszenzfärbung des Aktinzytoskeletts nach Expression des Intramers TR-M69 und verschiedener Kontrollen,
- Fig. 9: Fluoreszenzfärbung des Aktinzytoskeletts nach Expression des Intramers TR-M69 und verschiedener Kontrollproteine;
- Fig. 10: das Prinzip der Herstellung eines Aptazyms auf Basis einer der erfindungsgemäßen Nukleinsäuren; und
- Fig. 11: eine Pol III-Expressionskassette.

Fig. 1 zeigt eine schematische Darstellung des Aktivierungs- und Deaktivierungszyklus von ARF-Proteinen. ARF-Proteine gehören zu einer Familie von regulatorischen GTPasen, die auch als G-Proteine bezeichnet werden. ARF-Proteine sind an der Regulierung der Membranorganisation in eukaryontischen Zellen beteiligt und werden sowohl in einzelligen Organismen, wie Hefen, als auch in höheren Eukaryonten, wie Pflanzen und Tieren, gefunden. Ist GDP an die ARF-Proteine gebunden sind diese inaktiv. Eine Gruppe von Proteinen, die sogenannten GEF-("guanosine nucleotid exchange factors")-Proteine katalysieren den Austausch von GDP gegen GTP und aktivieren so die ARF-Proteine. Die Gruppe der für ARF-Proteine spezifischen GEF-Proteine wird hierin als ARF-GEF-Proteine bezeichnet. Eine andere Gruppe von Proteinen, die sogenannten GAP-("GTPase activating protein")-Proteine induzieren die GTPase-Aktivität der ARF-Proteine. Nach Freisetzung von Orthophosphat (Pi) befinden sich die ARF-Proteine wieder in dem GDP-gebundenen, inaktiven Zustand.

Fig. 2 zeigt eine Übersicht über verschiedene Mitglieder der ARF-GEF-Proteine (nach Donaldson und Jackson, Curr. Opin. Struct. Biol. 12 (2000), 475-482). Die schwarze Box stellt die Sec7-Domäne dar, die für die GDP/GTP-Austauschaktivität verantwortlich ist. Die DCB-Domäne (gepunktet) vermittelt die und die Bindung von Cyclophillinen. Die CC-(coiled coil)- und PH-(pleckstrin homology)-Domänen der kleinen ARF-GEFs sind schräg und längs gestreift dargestellt. Bei EFA6 handelt es sich um ein neues Protein, das verschieden ist von Cytohesin. Es gehört zwar zur Familie der ARF-GEF-Proteine, gehört aber weder zur Gruppe der großen, noch zur Gruppe der kleinen ARF-GEF's ( wie Cytohesin 1, 2, 3 und 4). Es ist jedoch Brefeldin A-sensitiv, weist ein Molekulargewicht von etwa 70 kD auf und enthält zudem noch prolinreiche Motive. Die Sensitivität bzw. Resistenz gegenüber Brefeldin A ist angegeben.

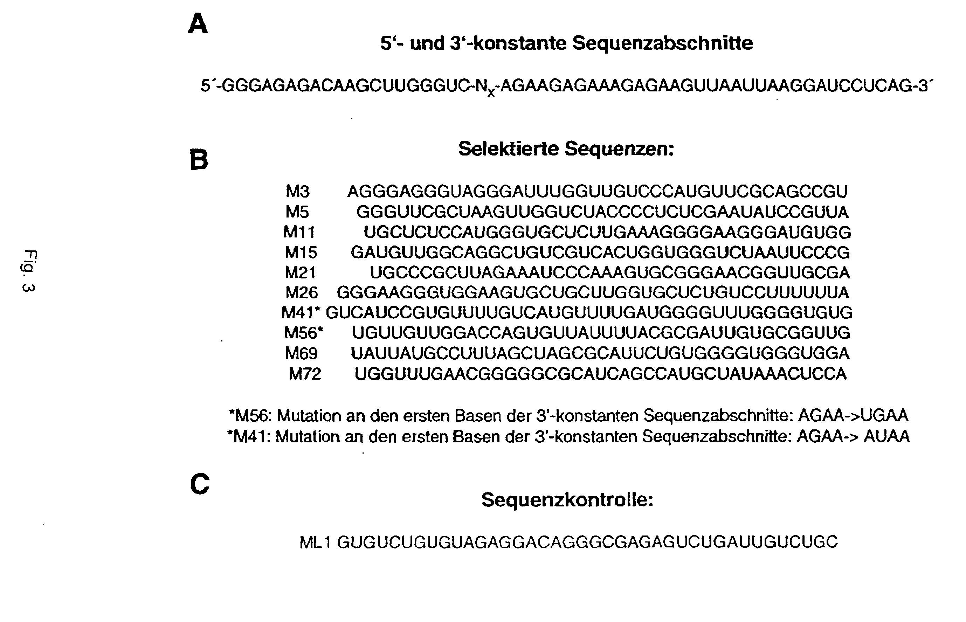

Fig. 3 zeigt den grundsätzlichen Aufbau der Bibliothek aus Nukleinsäuresequenzen, die für die in vitro Selektion verwendet wurde, sowie die Sequenzen der durch die in vitro Selektion erhaltenen Aptamere gegen Cytohesin-1. In a) sind die 5'- und 3'-konstanten Abschnitte der RNA-Bibliothek dargestellt, die die individuellen Sequenzen der RNA-Bibliothek (dargestellt durch Nₓ) flankieren. b) zeigt die individuellen, der Region Nₓ entsprechenden Sequenzen der Aptamere aus der in vitro Selektion gegen Cytohesin-1. Für die Sequenzen M56 und M41 (markiert durch *) sind Punktmutationen in den ersten Basen der 3'-konstanten Region angegeben, die bei der Sequenzierung der angereicherten RNA-Bibliothek aus Zyklus 11 identifiziert wurden. c) zeigt die Sequenz ML1, die Cytohesin-1 nicht bindet und analog den Sequenzen aus b) als Negativkontrolle in weiteren Experimenten verwendet wurde. Die Sequenz ML1 kann auch als Negativkontrolle in den hierin beschriebenen Kits verwendet werden.

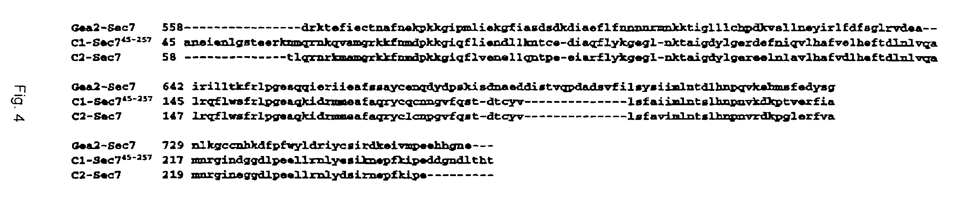

Fig. 4 zeigt den Vergleich der Aminosäuresequenz (in Einzelbuchstabenangabe) der Sec7-Domänen des hochmolekularen ARF-GEFs Gea2 (Gea2-Sec7) (Brefeldin A sensitiv), und der kleinen ARF-GEFs Cytohesin-1 (C1-Sec7) und Cytohesin-2 (C2-Sec7) (beide nicht Brefeldin A sensitiv), die targets der erfindungsgemäßen Nukleinsäuren sind. Die Aminosäurepositionen sind angegeben.

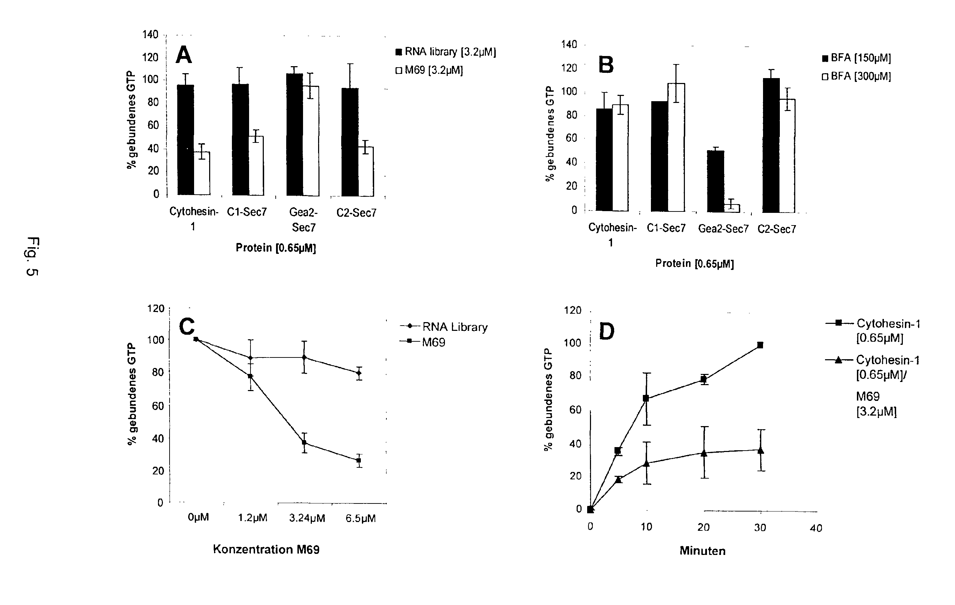

Fig. 5 zeigt die Daten der durch eine der erfindungsgemäßen Nukleinsäuren, die in dem in dieser Figur dargestellten Versuchen als Aptamer verwendet wurde, vermittelten in vitro Inhibition der GDP/GTP-Austauschaktivität mit ARF-1 als Substrat. A: GDP/GTP-Austauschaktivität von Cytohesin-1 (Vollängenprotein), C1-Sec7, C2-Sec7 und Gea2-Sec7 (vgl. Fig4) mit ARF-1 als Substrat in der Anwesenheit des Aptamers M69 (3,2 µM) (weiße Balken) und der unselektierten RNA-Bibliothek (3,2 µM) (schwarze Balken). Die Menge von an ARF-1 gebundenem γ³⁵S-GTP nach 30 Minuten in einem Experiment mit Cytohesin-1 ohne RNA wurde als 100%-Wert gesetzt. Deutlich ist zu sehen, das die Menge an γ³⁵S-GTP, das an ARF-1 gebunden wird bei dem kleinen ARF-GEF Cytohesin-1 oder den Sec7-Domänen C1-Sec7 und C2-Sec7 gebunden wird, in Anwesenheit des Aptamers M69 gegenüber der unspezifischen Kontrolle (weiße Balken) reduziert wird. Dagegen wird die durch die Sec7-Domäne des hochmolekularen ARF-GEF Gea2 (Gea2-Sec7) katalysierte Bindung von γ³⁵S-GTP an ARF-1 nicht beeinflusst. B: Sensitivität des GDP/GTP-Austauschs von Cytohesin-1, C1-Sec7, C2-Sec7 und Gea2-Sec7 gegenüber Brefeldin A. Während die Sec7-Domäne des hochmolekularen ARF-GEF's (Gea2-Sec7) deutlich durch Brefeldin A blockiert wird, hat dieser Inhibitor erwartungsgemäß keinen Einfluss auf Cytohesin-1, C1-Sec7 oder C2-Sec7. C: Konzentrationsabhängigkeit der Inhibition der GDP/GTP-Austauschaktivität von Cytohesin-1 an ARF-1 durch das Aptamer M69 (schwarze Quadrate) und der nicht-selektierten RNA-Bibliothek (schwarze Rhomben). Deutlich ist die Konzentrationsabhängigkeit von der Konzentration des Aptamerinhibitors M69 zu sehen, während auch ansteigende Konzentrationen der unspezifischen RNA-Bibliothek kaum Einfluss auf die Menge des an ARF-1 gebundenen γ³⁵S-GTP haben. D: Zeitverlauf der Inhibition der durch Cytohesin-1 vermittelten GDP/GTP-Austauschaktivität mit Aptamerinhibitor M69 (schwarze Dreiecke) und ohne Aptamer (schwarze Vierecke).

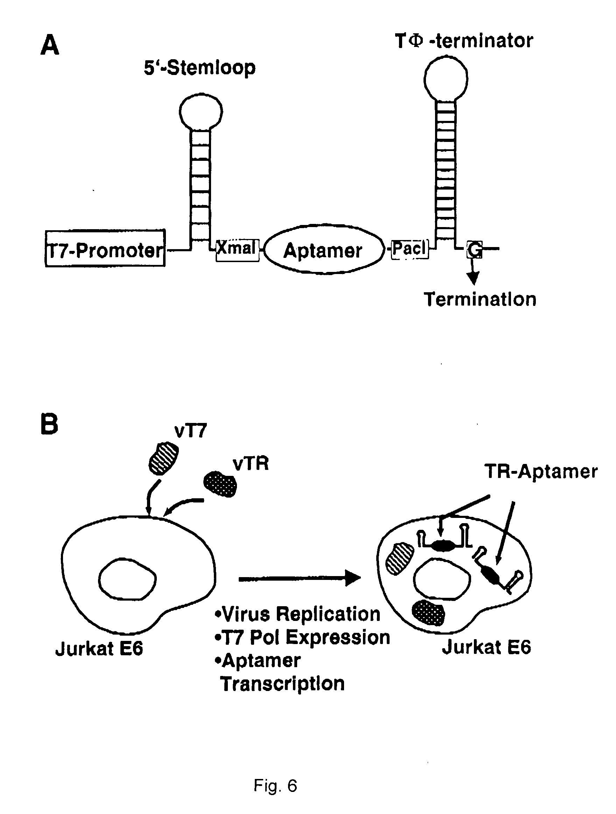

Fig. 6 zeigt das TR-Expressionssystem zur zytoplasmatischen Expression von intrazellulären Aptameren (Intrameren) mittels rekombinanten Vacciniaviren. Dieses Sytem ist ausführlich beschrieben in Blind et al., Proc. Natl,. Acad. Sci. USA 96 (1999), 3606-3609 und Blind et al., PCT/EP/00/02727) A: Die Expressionskassette TR. Das Aptamer wird über die Restriktionsschnittstellen Xma I und Pac I in die Expressionskassette kloniert, die unter der Kontrolle des T7-RNA-Polymerasepromotors steht. In dem Transkript wird die Aptamer-RNA von zwei Stemloopstrukturen (5'-Stemloop und TΦ-Terminator) flankiert. Diese zusätzlichen RNA-Sequenzen dienen der intrazellulären Stabilisierung und der korrekten Termination der Aptamere durch die T7-RNA-Polymerase. Die Aptamersequenzen in dem TR-Expressionskontext werden als TR-Intramere bezeichnet. B: Infektionsschema zur Expression der TR-Intramere. Zur Expression der TR-Intramere werden die Zielzellen (hier Jurkat-E6-Zellen, TIB 152, American Type Culture Collection (ATCC), Manassas, USA)) mit zwei rekombinanten Vacciniaviren infiziert. Der eine (vTR) trägt in seinem Genom die TR-Expressionskassette, der andere (vT7) exprimiert die T7-RNA-Polymerase unter der Kontrolle eines Vacciniapromotors. Nach Infektion der Zellen mit beiden Viren werden die TR-Expressionskassetten durch die gebildete T7-RNA-Polymerase transkribiert. Da der Lebenszyklus der Vacciniaviren exklusiv im Zytoplasma stattfindet wird auch die TR-Intramer-RNA dort exprimiert.

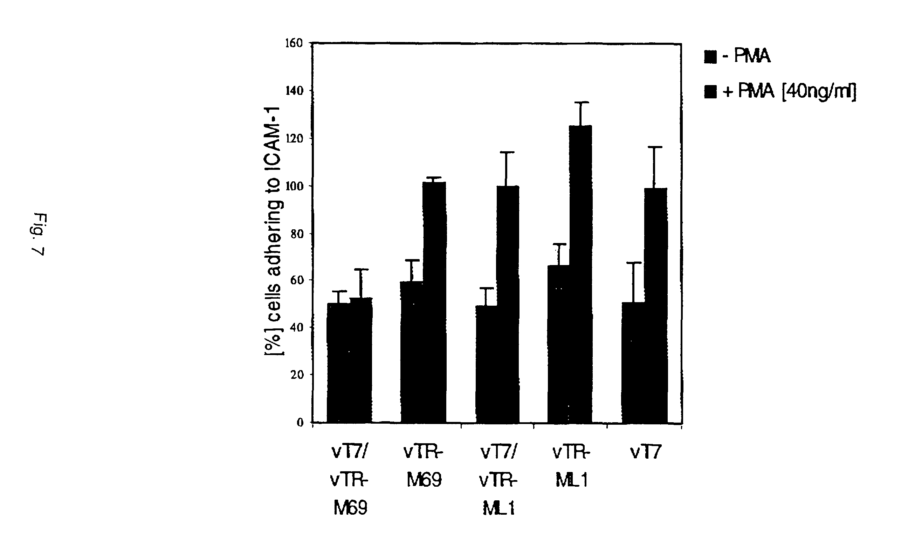

Fig. 7 zeigt die Inhibition der durch das Integrin LFA-1 vermittelten zellulären Adhäsion, wenn das Intramer TR-M69 in Jurkat E6-Zellen exprimiert wird (vgl. Fig 6). Die Adhäsion von Jurkat E6-Zellen an Petrischalen, die mit dem natürlichen Interaktor des LFA-1-Integrins ICAM-1 ("intercellular adhesion molecule-1") beschichtet wurden, kann durch Zugabe von Phorbolestern (PMA) stimuliert werden (stimulierte Werte: schwarze Balken; unstimulierte Werte: weiße Balken). Die Stimulierung lässt sich bei Einzelinfektion mit verschiedenen Kontrollvacciniaviren (vT7, vTR-ML1, vTR-M69) gut beobachten. Ebenso hat die Expression einer Kontrollsequenz (ML1, vgl. Fig. 3c) durch Doppelinfektion mit den rekombinanten Vacciniaviren vT7 und vTR-ML1 (vT7/vTR-ML1) keinen Effekt auf die stimulierte Adhäsion der Zellen. Als 100%-Wert wurde der Wert für die stimuliert Adhäsion von Zellen gesetzt, die zur Kontrolle nur mit dem rekombinanten Vacciniavirus vTR-M69 infiziert wurden. Wird jedoch das für die Sec7-Domäne von Cytohesin-1 spezifische Aptamer TR-M69 durch Doppelinfektion mit den Viren vT7 und vTR-M69 (vT7/vTR-M69) in Jurkat E6-Zellen exprimiert, wird die durch PMA induzierte stimulierte Adhäsion vollständig blockiert.

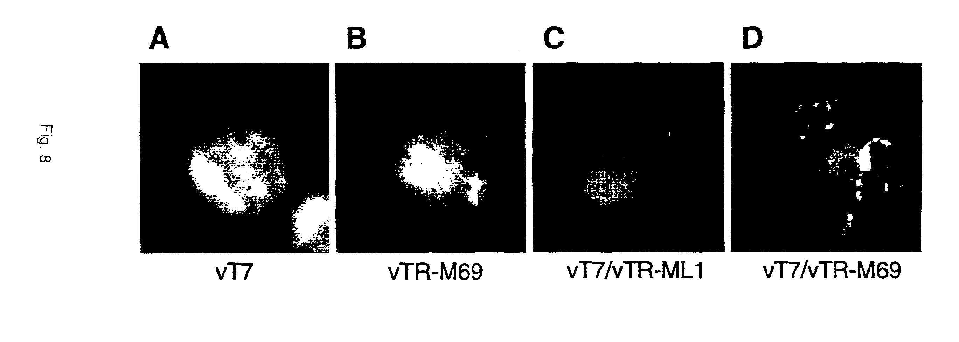

Fig 8 zeigt die Fluoreszenzfärbung des Aktinzytoskeletts nach der Infektion von Jurkat E6-Zellen mit rekombinanten Vacciniaviren. Die Jurkat E6-Zellen (TIB 152, American Type Culture Collection (ATCC), Manassas, USA ) wurden 5 Stunden nach der Infektion mit den im folgenden beschriebenen verschiedenen rekombinanten Vacciniaviren mit 40 ng/ml PMA für eine halbe Stunde bei 37°C stimuliert und adhärierten auf mit Fibronectin beschichteten Objektträgern. Nach Fixierung und Permeabilisierung wurde das Aktinzytoskelett mit TRITS-markiertem Phalloidin angefärbt. Die Visualisierung der Zellen erfolgte durch Fluoreszenzmikroskopie A: Einzelinfektion der Zellen mit einem rekombinanten Vacciniavirus, der die T7-RNA-Polymerase exprimiert (vT7). Die Expression der Polymerase allein führt zu einer normalen zellulären Morphologie und Organisation des durch TRITS-Phalloidin angefärbten Zytoskeletts. B: Infektion der Zellen mit einem rekombinanten Vacciniavirus, der die Expressionskassette für das intrazelluläre Aptamer (Intramer) TR-M69 in seinem Genom integriert trägt. Das TR-Intramer wird infolge des Fehlens einer geeigneten Polymerase, im vorliegenden Falle einer T7-Polymerase, nicht exprimiert und die Infektion interferiert nicht mit der Organisation des Zytoskeletts. C: Doppelinfektion der Zellen mit den rekombinanten Vacciniaviren vT7 und vTR-ML1. Die Expression der Kontrollsequenz TR-ML1 durch die Doppelinfektion mit vT7 und vTR-ML1 hat keinen Einfluss auf die Organisation des Zytoskeletts. D: Die Expression des Aptamers TR-M69 durch Doppelinfektion mit den rekombinanten Vacciniaviren vT7 und vTR-M69 stört die Strukturierung des Aktinzytoskeletts und bewirkt eine veränderte sternförmige Morphologie.

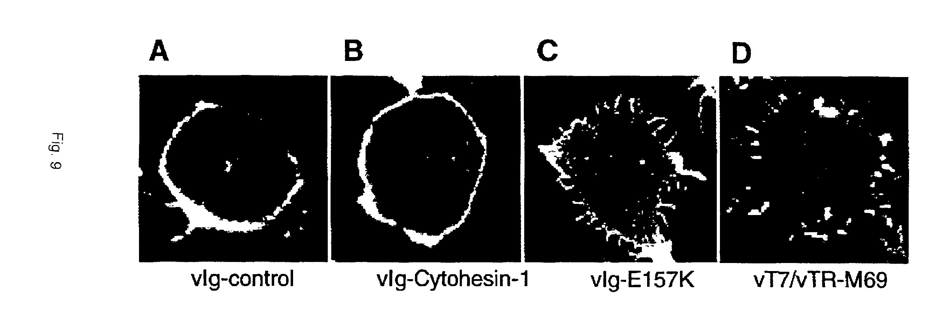

Fig. 9 zeigt die Fluoreszenzfärbung des Aktinzytoskeletts nach der Infektion von Jurkat E6-Zellen mit rekombinanten Vacciniaviren. Die nach Stimulierung mit PMA auf Fibronectin adhärierenden und mit TRITS-Phaloidin angefärbten Zellen wurden mittels konfokaler Lasermikroskopie untersucht. Gezeigt ist eine horizontale Sektion der jeweils analysierten Zellen. A: Infektion der Zellen mit einem Kontrollvirus, der rekombinante CH1-CH2-Domänen des humanen IgG-Immunglobulins exprimiert. Die Expression des IgG-Fragments zeigt keinen Effekt auf die Morphologie der Zellen. B: Infektion der Zellen mit einem rekombinanten Vacciniavirus, der humanes Cytohesin-1 als Fusionsprotein mit den CH1-CH2-Domänen des humanen IgG-Immunglobulins exprimiert. Die Expression von Cytohesin-1 interferiert nicht mit der Organisation des Zytoskeletts. C: Infektion der Zellen mit einem rekombinanten Vacciniavirus, der die mutierte Form von Cytohesin-1 (E157K), bei der Glutamt an Position gegen Lysin ausgetauscht ist wodurch die Mutante die GDP/GTP-Austauschaktivität verliert. als Fusionsprotein mit den CH1-CH2-Domänen des humanen IgG-Immunglobulins exprimiert. Die Infektion mit vE157K führt zu einer deutlichen Änderung in der Organisation des Aktinzytoskeletts. D: Die Expression des Aptamers TR-M69 durch Doppelinfektion mit den rekombinanten Vacciniaviren vT7 und vTR-M69 bewirkt die gleichen phänotypischen Veränderungen wie die Expression der mutanten Form von Cytohesin-1 (vE157K) (Fig 9c).

Fig: 10 zeigt das Prinzip der Herstellung eines Aptazyms auf Basis einer der erfindungsgemäßen Nukleinsäuren und die Detektion der Bindung der Sec7-Domäne an das Aptazymkonstrukt. A) Das Aptazym besteht aus einem allosterischen Zentrum, das von einer der erfindungsgemäßen Nukleinsäuren besteht (Aptamerdomäne). Dieses allosterische Zentrum ist über eine Bindegliedsequenz mit mit der Ribozymdomäne verbunden. In diesem Fall handelt es sich bei der Ribozymdomäne um das katalytische Zentrum eines sogenannten Hammerheadribozyms (HHR). Als Substrat für das Aptazym dient ein sogenanntes FRET-Oligonucleotid (Substrat: unterstrichene Buchstaben), das am 5'-Ende mit dem Fluoreszenzfarbstoff FAM (Donor: 6-Carboxy-Fluorescein) und am 3'-Ende mit dem Fluoreszenzfarbstoff TAMRA (Quencher: 6-Carboxy-Tetramethyl-Rhodamin) markiert ist. Derartige Oligonucleotide können synthetisch hergestellt werden und sind kommerziell erhältlich (z.B. über Eurogentec, Seraign, Belgien oder IBA, Göttingen, Deutschland). Das FRET-Oligonucleotid bindet über komplementäre Basenpaarung an die Ribozymdomäne. Nach dem für FRET-Assays gängigen Prinzip, das dem Fachmann auf diesem Gebiet geläufig ist (Hanne et al., Nucleosides and Nukleotides 17 (1998), 1835-1850; Singh et al., RNA 5, 1348-1356). Durch die räumliche Nähe des Donors zum Quencher kann wird die Fluoreszenzemission des Donors (hν': 516 nm) nach Anregung bei seiner Absorptionswellenlänge (hv: 496 nm) vom Quencher absorbiert. Dieser Effekt beruht auf der Tatsache, daß sich die Emissionswellenlänge des Donors mit der Absorptionswellenlänge des Quenchers überlagert. Es entsteht kein meßbares Signal. In der hier abbgebildeten Form ist das Aptazym inaktiv. B) Bindet die Sec7-Domäne an die Aptamerdomäne (allosterisches Zentrum) des Aptazyms induziert dies eine Konformationsänderung, die zur Aktivierung der Ribozymdomäne und zur Spaltung des Substrats führt. Nach Hydrolyse des FRET-Substrates durch das Ribozym können sich die Spaltstücke in Lösung voneinander entfernen. Die Fluoreszenz (Emission: hν') des Donors ist nun nicht mehr intramolekular durch den Quencher gelöscht, und das Ribozym steht für einen nächsten Katalysezyklus zur Verfügung. Die Aufhebung des FRET-Effektes bewirkt einen meßbaren Anstieg der FAM-spezifischen Fluoreszenz in der Probe. Dieser Effekt ist dem Fachmann bekannt und in der Literatur beschrieben (Jenne A. et al., Angew. Chem. 111 (1999), 1383-1386) Da der Anstieg der Fluoreszenz direkt proportional zur Spaltungsaktivität des Ribozyms ist, können durch die gemessenen Daten die Bindung der Sec 7-Domäne an das Aptazym nachgewiesen werden. Die hier abgebildeten Sequenzen des Substrats und der Ribozymdomäne sind als nicht limitierende Beispiele angeführt. Prinzipiell können andere, den Fachleuten auf diesem Gebiet bekannte Konstrukte aus Ribozymdomänen und Substraten oder Nachweismethoden der Ribozymaktivität verwendet werden (Übersichtsartikel: Soukup und Breaker, Curr. Opin. Struct. Biol. 10 (2000), Soukup und Breaker, Trends Biotechnol. 17 (1999), 469-76)

### Beispiel 1: Selektion von Cytohesin-bindenden Aptameren

RNA-Aptamere gegen Cytohesin-1 wurden durch in vitro Selektion aus einer Bibliothek von RNA-Molekülen mit einer randomisierten Region von 40 Nukleotiden selektiert. Die randomisierte Region wurde 5'- und 3'-seitig von 2 konstanten Bereichen flankiert, die während des Selektionsprozesses als Primerannealingsequenzen für die enzymatische Amplifikation der Nukleinsäuren durch reverse Transkription und die Polymerasekettenreaktion dienten (Vergleiche hierzu auch Fig. 3.). Zur Herstellung der Nukleinsäurebibliothek für die in vitro Selektion wurde ein ssDNA-Oligonukleotid mit der Sequenz 5'-CTATA GGGAG AGACA AGCTT GGGTC N₄₀ AGAAG AGAAA GAGAA GTTAA TTAAG GATCC TCAG-3' (hierin auch als SEQ ID No. 14 bezeichnet) (N₄₀ steht für 40 randomisierte Positionen mit einem ausgewogenem Verhältnis der vier Basen A,C,G und T von 1:1:1:1) auf einem Oligonukleotid-Syntheseapparat (Expedite, Millipore) nach dem Phosphoramiditverfahren synthetisiert wurden. Das einzelsträngige Oligonukleotid wurde durch PCR mit den Primern 5'-TCT AAT ACG ACT CAC TAT AGG GAG AGA CAA GCT TGG GTC-3' (entsprechend SEQ ID No. 12) und 5'-CTG AGG ATC CTT AAT TAA CTT CTC TTT CTC-3' (entsprechend SEQ ID No. 13) doppelstränig gemacht. Die Komplexität der RNA-Bibliothek lag bei etwa 10¹⁵ verschiedenen Sequenzen.

Die prinzipiellen Schritte des Selektionsprozesses sind ausführlich der Literatur beschrieben und den Fachleuten auf diesem Gebiet bekannt (Tuerk, C. und Gold, L., Science 249 (1990), 505-510; Ellington, A. D. und Szostak, J. W., Nature 346 (1990), 818-822; Nieuwlandt, D. et al., Biochemistry 34 (1995), 5651-5659; Conrad, R. et al. Methods Enzymol. 267 (1996)). Das Cytohesin-1-Protein wurde als Fusionsprotein mit einer konsekutiven Abfolge von sechs Histidin-Resten versehen, die auch als 6 x His-Tag (Cytohesin-1-His6) bezeichnet wird, in E.coli überexprimiert und durch Ni²⁺ -NTA Affinitätschromatographie aufgereinigt. Die Protokolle zur Expression und Aufreinigung von His-Tag-Proteinen sind etabliert und den Fachleuten bekannt (Kolanus et al., Cell 86 (1996), 233-242). 3,75 mg des gereinigten Cytohesin-1-Proteins wurden nach Angaben des Herstellers (Amersham Pharmacia Biotech) an CNBr-aktivierte Sepharose gekoppelt. Als Präselektionsmatrices wurde einerseits CNBr-aktivierte Sepharose mit 0,2 M Glycin geblockt, andererseits Lysate von *E. coli*-Zellen, die analog zu Cytohesin-1-His6 behandelt wurden. Wasch- und Elutionsfraktionen der reinen E.coli-Lysate an der Ni2+-NTA-Affinitätsmatrix wurden jeweils an CNBr-aktivierte Sepharose (Pharmacia) gekoppelt und in der späteren Präselektion verwendet. Um nicht-gebundene Proteine zu entfernen, wurden alle präparierten Sepharosen intensiv mit Puffer (137mM NaCI, 2.7mM KCI, 4.3mM Na₂HPO₄*7H₂O, 1.4mM KH₂PO₄) gewaschen und bei 4°C gelagert.

Für die erste Selektionsrunde wurden 1,4 nmol der oben beschriebenen dsDNA-Bibliothek als Templat für die in vitro Transkriptionsreaktion mit T7-RNA-Polymerase (Stratagene) nach den Angaben des Herstellers eingesetzt. Für alle folgenden Selektionszyklen wurde 250 pmol DNA als Templat verwendet. Die in vitro transkribierte RNA wurde durch Elektrophorese auf 8%-igen denaturierenden Polyacrylamidgelen gereinigt. Die Visualisierung der Nukleinsäurebanden erfolgte durch Fluoreszenzlöschung. Dazu wurden die präparativen Polyacrylamidgele in Klarsichtfolie verpackt, auf eine Dünnschichtchromatographie-Platte mit Fluoreszenzindikator (DC-Alufolie Kieselgel 60 F₂₅₄, Merck) gelegt und mit einer UV-Handleuchte bei einer Wellenlänge von 254 nm bestrahlt. Nukleinsäurebanden der richtigen Länge wurden mit einem sterilen Skalpell ausgeschnitten und durch die "Crush and Soak"-Methode in 0,3 M Natriumacetat eluiert. Nach Präzipitation der Nukleinsäuren wurde die RNA in sterilem H₂O aufgenommen. Zur Selektion wurde die RNA-Bibliothek in unterschiedlichen Konzentrationen (vgl. Tabelle 1) in Selektionspuffer (3mM MgCl₂, 137mM NaCI, 2.7mM KCI, 4.3mM Na₂HPO₄*7H₂O, 1.4mM KH₂PO₄, 4mM DTT, RNasin 0,8U/µL, pH 7,3) verdünnt und 1 Minute bei 95°C denaturiert. Zur Faltung der RNA wurde die Bibliothek 10 min bei 4°C inkubiert. Danach wurden die Protein-Sepharosen zugegeben. Die genauen Reaktionsbedingungen sind in der folgenden Tabelle 1 dargestellt.

**Tabelle 1:**

| Parameter der dreizehn Zyklen des in vitro Selektionsexperiments gegen Cytohesin-1. | | | | | | | | |
|---|---|---|---|---|---|---|---|---|
| **Selektionszyklus** | **Säulenvolumen** | **Präselektion** | **RNA (pmole)** | **Inkubations volumen** | **Waschvdumen** | **Butionsvolumen** | **Kompetitor** | **Selektionszyklus** |
| 1 | 100 | - | 20000 | 2000 | 1000 | 1500 (Urea/EDTA) | - | 199 |
| 2 | 50 | Glycin/100 | 1000 | 412 | 600 | 400 (U/E)/200 (ELU) | - | 98 |
| 3 | 25 | Glycin/50 | 500 | 466 | 800 | 400 (ELU) | - | 47 |
| 4 | 25 | Ecoli E/50 | 500 | 466 | 900 | 400(ELU) | - | 46 |
| 5 | 25 | Ecoli W50 | 500 | 466 | 900 | 400 (ELU) | - | 45 |
| 6 | 25 | Ecoli W50 | 500 | 466 | 900 | 400(ELU) | - | 44 |
| 7 | 25 | Ecoli W25 | 500 | 466 | 900 | 600 (ELU) | - | 43 |
| 8 | 25 | Ecoli W25 | 250 | 466 | 900 | 400(ELU) | - | 42 |
| 9 | 25 | Ecoli E/25 | 250 | 466 | 1000 | 400 (ELU) | - | 41 |
| 10 | 25 | Ecoli E/25 | 250 | 466 | 1000 | 400(ELU) | - | 40 |
| 11 | 25 | Ecoli E/25 | 250 | 170 | 1600 | 600 (ELU) | 2.5 ug/ul Heparin | 39 |
| 12 | 25 | Ecoli E/25 | 250 | 466 | 47000 | 400 (ELU) | - | 38 |
| 13 | 5 | Ecoli E/25 | 250 | 423 | 8400 | 400 (ELU) | 1.2 ug/ul Heparin | -3 |

Die Bindungsreaktionen erfolgten bei 37°C für 1 h. Danach wurde die Sepharose intensiv mit Selektionspuffer gewaschen, um alle nicht gebundenen RNA-Sequenzen zu entfernen. Gebundene RNA-Moleküle wurden durch Waschen mit denaturierendem Puffer (30mM Tris pH 6.8, 20% Glycerin, 2% SDS, 1M DTT) isoliert, mit Phenol und Chloroform extrahiert und durch Ethanolpräzipitation gefällt. Die RNA wurde mit Tth DNA Polymerase nach Angaben des Herstellers (Roche) revers transkribiert und mit DAp DNA Polymerase (Eurogentec) durch Polymerasekettenreaktion (PCR) amplifiziert. Die PCR-DNA diente als Templat für die anschließende in vitro Transkription. Präselektionen wurden beginnend mit dem zweiten Selektionszyklus entweder mit Glycin-Sepharose oder E.coli-Lysat-Sepharose durchgeführt. Von Zyklus 11 bis 13 wurde dem Selektionspuffer zur Erhöhung der Stringenz Heparin in einer Konzentration von 1µg/ml zugegeben. Nach der dreizehnten Selektionsrunde wurde die Nukleinsäurebibliothek kloniert und 72 Sequenzen analysiert. Unter den 72 Isolaten konnten zehn unterschiedliche Sequenzen identifiziert werden (vgl. Abbildung 3). Die häufigsten gefundenen Sequenzen waren die Klone M69 (61%) und M3 (18%). Die übrigen Sequenzen waren mit niedriger Frequenz repräsentiert. Die Sequenzen dieser zehn Klone entsprechen den SEQ ID No. 1 bis 10 und wurden hierin wie folgt zugeordnet:
SEQ ID No. 1, hierin auch als M3 bezeichnet: 
5´-GGGAGAGACAAGCUUGGGUCAGGGAGGGUAGGAUUUGGUUGUCCCAUGUUCGCAGCCGU
AGAAGAGAAAGAGAAGUUAAUUAAGGAUCCUCAG-3´
SEQ ID No. 2, hierin auch als M5 bezeichnet: 
5´-GGGAGAGACAAGCUUGGGUCGGGUUCGCUAAGUUGGUCUACCCCUCUCGAAUAUCCGUUA
AGAAGAGAAAGAGAAGUUAAUUAAGGAUCCUCAG-3´
SEQ ID No. 3, hierin auch als M11 bezeichnet: 
5′-GGGAGAGACAAGCUUGGGUCUGCUCUCCAUGGGUGCUCUUGAAAGGGGAAGGGAUGUGGA
GAAGAGAAAGAGAAGUUAAUUAAGGAUCCUCAG-3′
SEQ ID No. 4, hierin auch als M15 bezeichnet: 
5´-GGGAGAGACAAGCUUGGGUCGAUGUUGGCAGGCUGUCGUCACUGGUGGGUCUAAUUCCCG
AGAAGAGAAAGAGAAGUUAAUUAAGGAUCCUCAG-3´
SEQ ID No. 5, hierin auch als M21 bezeichnet: 
5´-GGGAGAGACAAGCUUGGGUCUGCCCGCUUAGAAAUCCCAAAGUGCGGGAACGGUUGCGAA
GAAGAGAAAGAGAAGUUAAUUAAGGAUCCUCAG-3´
SEQ ID No. 6, hierin auch als M26 bezeichnet: 
5´-GGGAGAGACAAGCUUGGGUCGGAAGGGUGGAAGUGCUGCUUGGUGCUCUGUCCUUUUU
UAAGAAGAGAAAGAGAAGUUAAUUAAGGAUCCUCAG-3´
SEQ ID No. 7, hierin auch als M41 bezeichnet: 
5´-GGGAGAGACAAGCUUGGGUCGUCAUCCGUGUUUUGUCAUGUUUGAUGGGGUUUGGGGU
GUGAUAAGAGAAAGAGAAGUUAAUUAAGGAUCCUCAG-3´
SEQ ID No. 8, hierin auch als M56 bezeichnet: 
5´-GGGAGAGACAAGCUUGGGUCUGUUGUUGGACCAGUGUUAUUUACGCGAUUGUGCGGUUG
UGAAGAGAAAGAGAAGUUAAUUAAGGAUCCUCAG-3´
SEQ ID No. 9, hierin auch als M69 bezeichnet: 
5´-GGGAGAGACAAGCUUGGGUCUAUUAUGCCUUUAGCUAGCGCAUUCUGUGGGGUGGGUGGA
AGAAGAGAAAGAGAAGUUAAUUAAGGAUCCUCAG-3´
SEQ ID No. 10, hierin auch als M72 bezeichnet: 
5′-GGGAGAGACAAGCUUGGGUCUGGUUUGAACGGGGGCGCAUCAGCCAUGCUAUAAACUCCA
AGAAGAGAAAGAGAAGUUAAUUAAGGAUCCUCAG-3′
Kontrollsequenz, hierin auch als SEQ ID No. 11 oder ML1 bezeichnet:

5'-GGGAGAGACAAGCUUGGGUCGUGUCUGUGUAGAGGACAGGGCGAGAGUCUGAUUGUCUGC
AGAAGAGAAAGAGAAGUUAAUUAAGGAUCCUCAG-3'

### Beispiel 2: Bindungsspezifitäten der selektierten Aptamere

Um zu testen, ob die selektierten Aptamersequenzen auch natives Cytohesin-1 in Lösung erkennen können, wurden Filterbindungsexperimente durchgeführt. Die Interaktionsanalysen sind in der Literatur ausführlich beschrieben. (Jellinek et al., Proc. Natl. Acad. Sci. USA 90 (1993), 11227-11231; Jellinek et al., Biochemistry 33 (1994), 10450-10456).

Ansteigende Konzentrationen von Cytohesin-1-His6 (0,1 nM bis 500 nM) wurden mit den am 5'-Ende mit ³²P radioaktiv markierten erfindungsgemäßen Aptameren (1,0 nM) 1 h bei 37°C in Selektionspuffer inkubiert. Die RNA/Cytohesin-1-His6-Komplexe wurden anschließend über Nitrozelluloseacetatmischmembranen (HAWP 0,45 µm, Millipore) gefiltert. Die Filter wurden dann mit 5 ml Waschpuffer (3mM MgCl₂, 137mM NaCI, 2,7mM KCI, 4.3mM Na₂HPO₄*7H₂O, 1.4mM KH₂PO₄) gespült. Vor dem Experiment wurden die Filter 10 Minuten mit 40 mM 6-Aminocaprionsäure und 20% Ethanol vorbehandelt. Die auf den Filtern zurückgehaltene radioaktive RNA wurde durch Analyse auf einem Phosphorimager (Storm 860, Amersham) quantifiziert. Die Bestimmung der Dissoziationskonstanten (K_{d}) erfolgte wie bei Jellinek et al. beschrieben (Jellinek et al., Proc. Natl. Acad. Sci. USA 90 (1993), 11227-11231). Die zum Vergleich bestimmte K_{d} der unselektierten RNA-Bibliothek für das Cytohesin-1-His6-Protein lag bei > 4 µM. Von den analysierten Sequenzen band der Klon M69 Cytohesin-1-His6 mit einer Dissoziationskonstante von 16 nM (vgl. Tabelle 2). Als Kontrollen wurden gleiche Bindungsexperimente mit E. coli-Lysaten durchgeführt, die analog zu dem in E. coli exprimierten Cytohesin-1 behandelt wurden. Die vier getesteten Sequenzen (M56, M41, M5, M3) zeigten ebenfalls Bindung an Cytohesin-1.

Im nächsten Schritt wurde die Bindungsstelle des Aptamers M69 auf Cytohesin-1 näher eingegrenzt. Zu diesem Zweck wurden Filterbindungsstudien mit Subdomänenkonstrukten von Cytohesin-1 durchgeführt. Die PH-Domäne und die Sec7-Domäne von Cytohesin-1 wurden als 6xHis-Tag-Proteine (PH-His6 und C1-Sec7-His6) in E.coli überexprimiert und wie für Cytohesin-1-His6 beschrieben gereinigt. Das synthetische polybasische C-Peptid wurde von der Firma TopLab (München) bezogen. Die Sec7-Domäne des großen, Brefeldin A - sensitiven ARF-GEF's Gea2 (Gea2-Sec7) wurde als 6xHis-Tag-Protein von Cathy Jackson (Departement de Biologie Cellulaire et Moleculaire, CEA/Saclay, Frankreich) zur Verfügung gestellt. Die Bindungsstudien zeigten, dass die C1-Sec7-Domäne wie das Volllängenprotein durch M 69 mit einer K_{d} von 16nM gebunden wurde. Keine Interaktion konnte hingegen mit der PH-Domäne oder der Sec7-Domäne von Gea2 nachgewiesen werden (siehe Tabelle 2). Die Experimente zeigen, dass für die Bindung des Aptamers M69 die Aminosäuresequenz der Sec7-Domäne des kleinen ARF-GEF's Cytohesin-1 benötigt wird, und dass das Aptamer C1-Sec7 sehr spezifisch von der verwandten Sec7-Domäne des großen ARF-GEF's Gea2 unterscheiden kann. Das Aptamer M5 erkennt dagegen sowohl die Sec7-Domäne von Cytohesin-1 als auch von Gea2 mit annähernd gleicher Affinität. Die PH-Domäne von Cytohesin-1 wird um eine Größenordnung schlechter erkannt. Das Aptamer M56 dagegen bindet am besten an das Vollängenprotein Cytohesin-1 und kaum an die Sec7-Domäne von Gea2. Da sowohl die Sec7-Domäne als auch die PH-Domäne von Cytohesin-1 mit etwas verminderten Affinitäten gegenüber dem Vollängenprotein gebunden werden, scheint das Aptamer eine Bindungsstelle an Cytohesin-1 zu erkennen, die aus beiden Domänen zusammengesetzt ist. Die Sec7-Domänen von Gea2, Cytohesin-1 (C1) und Cytohesin-2 (C2) sind in Abbildung 4 dargestellt.

**Tabelle 2:**

| Affinitäten einiger Aptamere (M5, M56, M69) aus der Selektion gegen Cytohesin-1. Die Dissoziationskonstante (nM) wurde für Cytohesin-1 (Volllängenprotein), die Sec7-Domäne von Cytohesin-1 (C1-Sec7), die PH-Domäne von Cytohesin-1 (C1-PH) und die Sec7-Domäne von Gea2 (G2-Sec7) ermittelt. Falls keine Interaktion messbar war, ist dies durch (-) angegeben. Alle Angaben zur Dissoziationskonstanten K_{d} erfolgen in nM. | | | | |
|---|---|---|---|---|
| | Cytohesin-1 | C1-Sec7 | C1-PH | G2-Sec7 |
| Aptamer M5 | 40,0 ±8,0 | 36,0 ±3,4 | 480±50 | 48,0 ±5,5 |
| M56 | 5,0±1,0 | 280±28 | 150±8,0 | > 1000 |
| M69 | 16,0±0,4 | 16,0±0,3 | - | - |

### Beispiel 3: Spezifische Inhibition des Guaninnukleotidaustauschs an ARF-1

Der Austausch von GDP gegen GTP aktiviert die ADP-Ribosylierungsfaktoren (ARF's). Nachdem gezeigt wurde, dass das Aptamer M69 nur die Sec7-Domäne des kleinen ARF-GEF's spezifisch erkennt, wurde der Einfluss des Aptamers auf dessen GDP/GTP-Austauschfunktion untersucht. Zu diesem Zweck wurde der Effekt des RNA-Moleküls M69 auf den Austausch von GDP gegen GTP an dem ARF1-Protein in Gegenwart von radioaktivem [γ³⁵S]-GTP in vitro untersucht.

Ein chimäres ARF-1-Protein, an das am Carboxyterminus die konstanten CH1- und CH-2-Domänen des humanem IgG-Immunglobuins zu Aufreinigungszwecken fusioniert wurden, wurde wie in der Literatur beschrieben in COS- oder CV-1-Zellen durch Infektion mit rekombinanten Vacciniaviren überexprimiert (Knorr et al., Eur. J. Biochem. 267 (2000), 3784-3791). Die Zellen wurden mit einem Zellschaber geerntet und zweimal mit PBS gewaschen. Danach wurden die Zellen mit Lysispuffer (100mM Tris (pH 8,0), 150mM NaCI, 2mM EDTA, 1% (v/v) Triton X-100, 10µg/mL Leupeptin, 1mM Phenylmethylsulfonylfluoride) aufgeschlossen und bei 20 000 g und 4°C für 4 min abzentrifugiert. Die Überstände wurden mit Protein A-Sepharose 6MB (Amersham Pharmacia Biotech) 2 h bei 4°C inkubiert. Die Sepharose wurden zweimal mit 1ml Lysispuffer gewaschen und in 50 mM HEPES äquilibriert. Nach vollständiger Entfernung des Überstands wurde die Sepharose in Puffer E (50mM HEPES (pH 7.4), 1mM MgCl₂, 1mM DTT, RNasin 0,8U/µl) äquilibriert. Vor der Zugabe von ARF-1 wurden Cytohesin-1, C2-Sec7 oder Gea2-Sec7 mit renaturierter Aptamer-RNA bei 37°C in Puffer E inkubiert. Die Austauschreaktion wurde durch Zugabe von 0,02 µCi/µl [γ³⁵S]-GTP (NEN Life Sciences) gestartet. Nach Inkubation bei 37°C für 30 min wurde die Reaktion durch Zugabe von 1 ml Waschpuffer (20mM HEPES pH 7.4, 100mM NaCI, 10mM MgCl₂) gestoppt, die Protein A Sepharose weitere zweimal mit Waschpuffer gewaschen. Der Waschpuffer wurde vollständig entfernt und nach Zugabe von Scinzillationsflüssigkeit wurde die gebundene Radioaktivität (γ³⁵-S GTP) durch Messung an einem Scinzillationszähler bestimmt. Ebenso wurden alle Zeitverläufe bestimmt und zu unterschiedlichen Zeitpunkten gestoppt. Die Menge an gebundenem [γ³⁵S]-GTP in den Reaktionen ohne Aptamer-RNA wurde als 100%-Wert gesetzt. Als Negativkontrolle dienten Ansätze, in denen die unselektierte RNA-Bibliothek zugegeben wurde, die keinen Effekt auf die ARF-GEF-Aktivität aller untersuchten GEF's zeigte (Abbildung 5 a und c). Cytohesin-1 (0,65 µM) erhöhte die Bindung von [γ³⁵S]-GTP an ARF-1 verglichen mit der unkatalysierten Reaktion um das Zehnfache (Daten nicht gezeigt). Diese Ergebnisse stimmen mit den in der Literatur beschriebenen Werten gut überein (Knorr et al. Eur. J. Biochem. 267 (2000), 3784-3791). Die Experimente zeigten, dass das Aptamer M69 ein potenter Inhibitor des GTP/GDP-Austauschs an ARF-1 durch Cytohesin-1 oder der isolierten Cytohesin-1-Sec-7 Domäne (C1-Sec7) ist. Des weiteren wird auch die Austauschfunktion der Sec-7-Domäne (C2-Sec7) von Cytohesin-2 (ARNO) inhibiert (siehe Abbildung 5 a). Die Inhibition von Cytohesin-1 ist konzentrationsabhängig (Abbildung 5 c). Desweiteren wird der durch die Sec7-Domäne (G2-Sec7) des große GEF-Proteins Gea2 katalysierte Guaninnukleotidaustausch nicht beeinflusst (vgl. Abbildung 5 a). In Kontrollexperimenten mit Brefeldin A konnte gezeigt werden, dass in diesem Fall, wie erwartet, die kleinen Sec7-Proteine oder -Domänen nicht inhibiert wurden, wohl aber G2-Sec7 (siehe Abbildung 5 b). Die Daten zeigen, dass das erfindungsgemäße Aptamer M69 spezifisch in der Lage ist zwischen den großen und kleinen GEF-Proteinen zu unterscheiden und die katalytische Aktivität der kleinen GEF's zu inhibieren. Das Aptamer ist damit der erste spezifische Inhibitor der GDP/GTP-Austauschreaktion der Brefeldin A-resistenten kleinen GEF-Proteine.

### Beispiel 4: Inhibition der Adhäsion von Jurkat E6 Zellen an ICAM-1

Zur Expression des erfindungsgemäßen Aptamers M69 im Zytoplasma von Jurkat E6 Zellen wurde ein auf der Infektion der Zielzellen mit rekombinanten Vacciniaviren basierendes System gewählt (Blind et al., Proc Natl. Acad. Sci. USA 96 (1999), 3606-3610). Dabei wird die für das Aptamer kodierende DNA über die Restriktionsschnittstellen Xma1 und Pac1 in die TR-Expressionskassette des Transfervektors pTR kloniert (vgl. Abbildung 6 a). Die von einem T7-RNA-Polymerase-Promotor transkribierte Aptamer-RNA wird dabei von einem der zytoplasmatischen Stabilisierung dienenden 5'-Stemloop und dem der Termination der Transkripte dienenden Tφ-Terminator flankiert. Die so entstehenden Transkripte werden nachfolgend als - erfindungsgemäße - TR-Intramere bezeichnet (z.B. TR-M69). Die Herstellung rekombinanter Vacciniaviren erfolgte wie in der Literatur beschrieben (Ausubel et al., Current Protocols in Molecular Biology (1987), Wiley & Sons Inc., New York, USA). Dabei werden die in dem Transfervektor pTR von Segmenten des viralen Thymidinkinasegens (TK-Gen) flankierten TR-Expressionkassetten über homologe Rekombination in das TK-Gen von Wildtyp-Vacciniaviren integriert. Die Expression der TR-Intramere erfolgte nach einem in der Literatur beschriebenen Doppelinfektionsschema (Fuerst und Moss, J. Mol. Biol. 206 (1989), 333-348), das in Abbildung 6B dargestellt ist. Dabei werden eukaryontische Zellen einerseits mit dem das Gen für das TR-Intramer enthaltenden Virus (vTR) und gleichzeitig mit einem die T7-RNA-Polymerase exprimierenden Virus (vT7) infiziert. Nach Expression der Polymerase werden dann die TR-Intramere von dem T7-RNA-Polymerase-Promotor aus im Zytoplasma der infizierten Zellen transkribiert (Blind et al., Proc Natl. Acad. Sci. USA 96 (1999), 3606-3610). Für die Expression von RNA-Aptameren in Zellen können neben Vacciniaviren auch andere den Fachleuten bekannte Expressionssysteme wie Semliki Forest Viren, retrovirale Vektoren, Plasmide, RNA-Polymerase II und III gesteuerte Transkriptionseinheiten u.a. verwendet werden (Cheetham GM, et al., Curr Opin Struct Biol. 10 (2000); 117-123; Paule und White, Nucleic Acid Res. 28 (2000), 1283-1298; Shi H. et al., Proc. Natl. Acad. Sci USA 96 (1999), 10033-10038; Bramlage et al., Tibtech 16 (1998), 434-438; Chakrabarti S, et al., Biotechniques. 23 (1997), 1094-1097; Tubulekas et al., Gene 190, 191-195; Bertrand et al., RNA 3 (1997), 75-88 ; Rossi, Tibtech 13 (1995), 301-305;.)

5 Stunden nach der Doppelinfektion von Jurkat E6-Zellen mit den Viren vT7 und vTR-M69 (ca. 10 pfu/Zelle) konnten Expressionsniveaus von ca. 100 000 TR-Intramermolekülen pro Zelle erreicht werden. Wie auch schon in früheren Arbeiten nachgewiesen (Blind et al., Proc Natl. Acad. Sci. USA 96 (1999), 3606-3610) führt nur die Doppelinfektion mit dem für die T7-RNA-Polymerase kodierenden Virus (vT7) und dem die Expressionskassette enthaltenden Virus (vTR-Intramer) zur Transkription der gewünschten RNA-Moleküle im Zytoplasma. Bei der Infektion mit nur einem einzelnen rekombinanten Vacciniavirus konnten keine nachweisbaren Mengen an transkribierter RNA nachgewiesen werden. Alle Experimente in Jurkat E6-Zellen wurden in den folgenden Experimenten 5 Stunden nach der Infektion mit den rekombinanten Vacciniaviren durchgeführt.

Die Sec7-Domäne von Cytohesin-1 stimuliert die Adhäsion von Leukozyten sowohl durch die direkte Interaktion mit der β2-Untereinheit von Integrinen als auch durch seine ARF-GEF-Aktivität (Geiger, C. et al., EMBO J. 19 (2000), 2525-2536). Adhäsionsexperimente wurden wie schon früher beschrieben in einer Leukozytenzelllinie (Jurkat E6-Zellen, TIB 152, American Type Culture Collection (ATCC), Manassas, USA), in denen die Aktivierung der b2-Integrine gut nachvollzogen werden kann, durchgeführt (Kolanus, W., et al., Cell 86 (1996), 233-242). 5 h nach der Infektion mit den rekombinanten Viren wurden die Zellen mit 40 ng/ml PMA (Phorbol-12-myristat-13-acetat), einem bekannten Induktor der Aktivierung von β2-Integrinen, 30 min bei 37°C inkubiert. Der Nachweis der stimulierten Adhäsion erfolgte wie schon früher beschrieben durch die Inkubation der Zellen auf mit dem natürlichen Liganden (ICAM-1) des β2-Integrins LFA-1 beschichteten Mikrotiterplatten (Nagel, W. et al., J. Biol. Chem. 273 (1998), 14853-14861).

Wie in Abbildung 7 gezeigt, konnte die Adhäsion der Zellen durch Zugabe von PMA (schwarze Balken) gegenüber der unstimulierten Hintergrundadhäsion (weiße Balken) deutlich hochreguliert werden. Alle Kontrollen, in denen die Jurkat E6-Zellen nur mit einem Virus infiziert wurden (vTR-M69, vTR-ML1, vT7) zeigten gute Stimulierbarkeit. Ebenso hatte die Expression einer Cytohesin-1 nicht-bindenden Kontrollsequenz TR-ML1 (Doppelinfektion: vT7/vTR-ML1) keinen Einfluss auf die Stimulierbarkeit. Dagegen wurde die stimulierte Adhäsion bei Expression des die Sec-7 Domäne bindenden erfindungsgemäßen Aptamers TR-M69 (Doppelinfektion: vT7/vTR-M69) nahezu vollständig blockiert (vgl. Abbildung 7).

Wie in früheren Arbeiten gezeigt wurde, kann eine Punktmutante von Cytohesin-1 (bezeichnet als E157K) den in vitro Guaninnukleotidaustausch an ARF-Proteinen nicht mehr katalysieren (Knorr, T. et al., Eur. J. Biochem. 267, 3784-3791). Überexpression dieser mutanten Form in Jurkat E6-Zellen resultiert in einer starken Inhibition der zellulären Adhäsion an ICAM-1. In Übereinstimmung mit diesen Daten zeigen die oben aufgeführten Experimente, dass das Aptamer M69 als Inhibitor der ARF-GEF-Funktion in vitro auch die Adhäsion in vivo blockieren kann. Das Aptamer ist daher der erste spezifische Inhibitor der entwickelt werden konnten und der auch in Zellkultur gegen die kleinen, gegen Brefeldin A resistenten ARF-GEF-Proteine aktiv ist.

### Beispiels: TR-M69 und Cytohesin-1 (E157K) induzieren Veränderungen des zellulären Aktinzytoskeletts

Bislang war sowohl über die mechanistische Rolle der GEF-Aktivität von Cytohesin-1 als auch das in vivo Substrat wenig bekannt. Zwei mögliche Funktionen wurden bislang diskutiert: Der Transport intrazellulärer Vesikel oder die Restrukturierung des an der Zytoplasmamembran gelegenen Aktinzytoskeletts. Frühere Arbeiten zeigten, dass die Überexpression von Cytohesin-1 in Jurkat E6-Zellen die Ausbreitung der Zellen auf mit ICAM-1 beschichteten Oberflächen induzierte (Geiger, C. et al., EMBO J. 19 (2000), 2525-2536). Dagegen konnte dieser Effekt bei der Überexpression der mutanten Form von Cytohesin-1 (E157K) nicht beobachtet werden. Um die Rolle der ARF-GEF-Aktivität von Cytohesin-1 besser zu charakterisieren wurden nun der erfindungsgemäße Aptamerinhibitor TR-M69 eingesetzt und die Effekte auf die Restrukturierung des Aktinzytoskeletts untersucht.

Dazu wurden Jurkat E6-Zellen wie beschrieben mit den rekombinanten Viren infiziert und nach 4,5 h mit 40 ng/ml PMA für 30 min bei 37°C stimuliert. Nach Zentrifugation der Zellen und Verwerfen des Überstands wurden die Zellen in HBSS ("Hanks buffered saline solution") aufgenommen und auf Objekträger aufgebracht, die zuvor mit Fibronektin beschichtet worden waren. Hierfür wurden die Objektträger bei Raumtemperatur für 2 h in Fibronektinlösung inkubiert und zweimal mit HBSS gewaschen. Die Jurkat E6-Zellen wurden dann für 30 min bei 37°C auf den Objekträgern belassen und ungebundene Zellen vorsichtig mit 2x 100 µl HBSS abgewaschen. Die adhärierenden Zellen wurden dann auf dem Objektträger mit frisch angesetztem 2%-igen Formaldehyd (v/v) in PBS bei 4°C über Nacht fixiert. Nach Inkubation in 2%-igem Glycin (w/v) in PBS für 2 h wurden die Zellen mit 0,2-%igem Triton X-100 (v/v) 10 min bei Raumtemperatur permeabilisiert. Zur Visualisierung des Aktinzytoskelletts wurden die Zellen mit TRITS-markiertem Phalloidin (Sigma-Aldrich Chemie GmbH, Deisenhofen, Deutschland) 1h bei Raumtemperatur gefärbt. Der Farbstoff bindet an Aktinfilamente und wird zur fluoreszenten Darstellung des Aktin-Zytoskeletts verwendet (Faulstich et al., Exp Cell Res 144, (1983), 73) Der überschüssige Fluoreszenzfarbstoff wurde mit zweimal 100 µl PBS abgewaschen und nach Aufbringen von "Mounting Medium" (Vector Laboratories, Burlingame, CA) die präparierten Zellen mit einem konfokalen Lasermikroskop oder mit einem Fluoreszenzmikroskop untersucht. Zielzellen, die einfach mit Kontrollviren (vT7 oder vTR-M69) infizierten wurden, zeigten einen normalen Adhäsionsphänotyp (Abb. 8a und b), der auch bei nicht infizierten Leukozyten gefunden wird (Daten nicht gezeigt). Ebenso hatte die Expression der Kontrollsequenz TR-ML1 durch die Doppelinfektion mit den rekombinanten Vacciniaviren vT7 und vTR-M69 keinen Effekt auf die Struktur des Aktinzytoskeletts (Abb. 8 C). Wurde jedoch der GEF-Inhibitor TR-M69 in den Jurkat E6-Zellen exprimiert (vT7/vTR-M69), wurde die Reorganisation des Zytoskellets deutlich gestört (Abb. 8 D und 9D).

Gleichzeitig wurde der Effekt der Expression des mutierten Cytohesin-1 (vlg-E157K) auf das Aktinzytoskelett untersucht. Zur Überexpression der Proteine erfolgte wie schon früher beschrieben (Kolanus et al., Cell 86 (1996), 233-242) durch rekombinate Vacciniaviren. Cytohesin-1 und E157K wurden als Fusionsproteine mit den konstanten CH2- und CH3-Domänen des humanen IgG-Immunglobulins hergestellt. Wie in Abbildung 9C gezeigt, induziert die Überexpression des mutanten Cytohesin-1 (vlg-E157K) den gleichen Phänotyp wie die Expression des erfindungsgemäßen Aptamers TR-M69 (Abbildung 9D). Die Überexpression von Cytohesin-1 (vlg-Cytohesin-1) resultiert in einem normalen Phänotyp (Abbildung 9B). Ebenso hatte die Expression eines Kontrollproteins (vlgcontrol) keinen nachweisbaren Effekt (Abbildung 9A). Die Ergebnisse belegen deutlich, dass der in vitro Selektionsprozess die spezifischen Inhibitoren der ARF-GEF-Aktivität der kleinen GEF-Proteine hervorgebracht hat. Neben der in vitro Inhibition der GEF-Funktion von Cytohesin-1 und -2 (ARNO) inhibierte die Expression des Aptamers die Adhäsion von Jurkat E6-Zellen an ICAM-1 und veränderte die Restrukturierung des Aktinzytoskeletts bei der Adhäsion an Fibronektin. Damit konnte erstmals direkt gezeigt werden, dass die Inhibition der GEF-Aktivität des endogenen Cytohesins die wichtige, regulative Anheftung von Leukozyten an den extrazellulären Liganden ICAM-1 blockiert, was einen neuen Ansatzpunkt zur Entwicklung spezifischerer, immunsuppressiver Medikamente darstellen.

Die in der vorstehenden Beschreibung, den Ansprüchen, dem Sequenzprotokoll sowie den Zeichnungen offenbarten Merkmale der Erfindung können sowohl einzeln als auch in beliebiger Kombination für die Verwirklichung der Erfindung in ihren verschiedenen Ausführungsformen wesentlich sein.

## Patentansprüche

1. Nukleinsäure, insbesondere eine isolierte Nukleinsäure, die in der Lage ist an einen Guaninnukleotid-Austauschfaktor für ADP-Ribosylierungsfaktoren zu binden und deren Derivate.

2. Nukleinsäure, die in der Lage ist an einen Guaninnukleotid-Austauschfaktor für ADP-Ribosylierungsfaktoren zu binden, **dadurch gekennzeichnet, dass** die Nukleinsäure eine Sequenz umfaßt, die ausgewählt ist aus der Gruppe, die SEQ ID No. 1, SEQ ID No. 2, SEQ ID No. 3, SEQ ID No. 4, SEQ ID No. 5, SEQ ID No. 6, SEQ ID No. 7, SEQ ID No. 8, SEQ ID No. 9 und SEQ ID No. 10 sowie deren jeweilige Derivate umfaßt.

3. Nukleinsäure nach Anspruch 1 oder 2, **dadurch gekennzeichnet, dass** der Guaninnukleotidaustauschfaktor zur Gruppe der kleinen Guaninnukleotidaustauschfaktoren für ARF-Proteine gehört, insbesondere zur Gruppe der Guaninnukleotidaustauschfaktoren, deren Molekulargewicht etwa 50 kDa oder weniger beträgt.

4. Nukleinsäure nach einem der Ansprüche 1 bis 3, **dadurch gekennzeichnet, dass** der Guaninnukleotidaustauschfaktor nicht durch Brefeldin A inhibiert wird.

5. Nukleinsäure nach einem der Ansprüche 1 bis 4, **dadurch gekennzeichnet, dass** der Guaninnukleotidaustauschfaktor Cytohesin-1 oder Cytohesin-2 und insbesondere die Sec7-Domäne von Cytohesin-1 oder Cytohesin-2 ist.

6. Nukleinsäure nach einem der Ansprüche 1 bis 5, **dadurch gekennzeichnet, dass** der Guaninnukleotidaustauschfaktor die Sec7-Domäne ist.

7. Nukleinsäure nach einem der Ansprüche 1 bis 6, **dadurch gekennzeichnet, dass** die Nukleinsäure eine solche ist, die ausgewählt ist aus der Gruppe, die DNA, RNA, Polynukleotide, Oligonukleotide, Aptamer, Aptazyme und Intramere umfaßt.

8. Vektor, bevorzugterweise ein Expressionsvektor, umfassend eine Nukleinsäure nach einem der Ansprüche 1 bis 7.

9. Zelle umfassend eine Nukleinsäure nach einem der Ansprüche 1 bis 7 und/oder einen Vektor nach Anspruch 8.

10. Zelle nach Anspruch 9, **dadurch gekennzeichnet, dass** die Zelle eine eukaryontische Zelle, bevorzugterweise eine tierische Zelle und ganz bevorzugt eine Zelle eines Säugetiers ist.

11. Zelle nach Anspruch 9 oder 10, **dadurch gekennzeichnet, dass** die Zelle ausgewählt ist aus der Gruppe, die Saccharomyces cerevisiae und C. elegans umfasst.

12. Tier, bevorzugterweise ein transgenes Tier, umfassend mindestens eine Zelle nach einem der Ansprüche 9 bis 11.

13. Verwendung einer Nukleinsäure nach einem der Ansprüche 1 bis 7 zur Herstellung eines Medikamentes.

14. Verwendung nach Anspruch 13, **dadurch gekennzeichnet, dass** das Medikament für die Behandlung von Erkrankungen ist, die ausgewählt sind aus der Gruppe, die Metastasierung von Lymphomen oder Melanomen, Autoimmunerkrankungen, Abstoßungsreaktionen, akute und chronische Entzündungen, Reperfusionsschäden, Transplantationserkrankungen, insbesondere Abstoßungsreaktionen bei Organtransplantationen und graft-vs-host-Erkrankungen umfasst.

15. Verwendung nach Anspruch 13 oder 14, **dadurch gekennzeichnet, dass** das Medikament die durch β-2 - Integrine vermittelte Adhäsion von Immunzellen beeinflußt.

16. Verwendung einer Nukleinsäure nach einem der Ansprüche 1 bis 7 und/oder eines Vektors nach Anspruch 8 in der Gentherapie.

17. Verwendung einer Nukleinsäure nach einem der Ansprüche 1 bis 7 zum Nachweis eines Guaninnukleotidaustauschfaktors

18. Verwendung einer Nukleinsäure nach einem der Ansprüche 1 bis 7 zur Komplexbildung mit einem Guaninnukleotidaustauschfaktor

19. Zusammensetzung, insbesondere pharmazeutische Zusammensetzung, umfassen eine Nukleinsäure nach einem der Ansprüche 1 bis 7, einen Vektor nach Anspruch 8 und/oder eine Zelle nach einem der Ansprüche 9 bis 11 zusammen mit einem geeigneten Trägermaterial.

20. Inhibitor für einen Guaninnukleotidaustauschfaktor, wobei der Guaninnukleotidaustauschfaktor zur Gruppe der kleinen Guaninnukleotidaustauschfaktoren für ARF-Proteine gehört, insbesondere zur Gruppe der Guaninnukleotidaustauschfaktoren, deren Molekulargewicht etwa 50 kDa oder weniger beträgt.

21. Inhibitor nach Anspruch 20, **dadurch gekennzeichnet, dass** der Guaninnukleotidaustauschfaktor nicht durch Brefeldin A inhibiert wird.

22. Inhibitor nach Anspruch 20 oder 21, wobei der Inhibitor eine Nukleinsäure nach einem der Ansprüche 1 bis 7 und/oder einen Vektor nach Anspruch 8 umfaßt.

23. Verfahren zum Screenen von Verbindungen, die die Wechselwirkung zwischen einem Guaninnukleotid-Austauschfaktor und einer Nukleinsäure nach den Ansprüchen 1 bis 7 inhibieren, insbesondere durch ein Verfahren, das mit Hochdurchsatzverfahren kompatibel ist und durch folgende Schritte gekennzeichnet ist:
a) Bereitstellen des Guaninnukleotid-Austauschfaktors und der Nukleinsäure
b) optional Bestimmen, ob eine Wechselwirkung zwischen dem Guaninnukleotid-Austauschfaktor und dem β-2-Integrin erfolgt,
c) Hinzufügen einer Kandidaten-Verbindung , und
d) Bestimmen, ob eine Wechselwirkung zwischen dem Guaninnukleotid-Austauschfaktor und der Nukleinsäure, bevorzugterweise durch die Kandidaten-Verbindung, inhibiert wird.

24. Verfahren nach Anspruch 23, **dadurch gekennzeichnet, dass** als weiterer Schritt vorgesehen ist Bestimmen, ob die identifizierte Verbindung die Guaninnukleotid-Austauschfunktion des Guaninnukleotid-Austauschfaktors für ein monomeres G-Protein inhibiert.

25. Verfahren nach Anspruch 23, **dadurch gekennzeichnet, dass** als weiterer Schritt vorgesehen ist Bestimmen, ob die Interaktion eines Guaninnukleotid-Austauschfaktors mit einem Integrin, bevorzugterweise die Wechselwirkung zwischen der Sec-7-Domäne von Cytohesin-1 und der β-2-Integrinuntereinheit inhibiert wird

26. Verfahren nach Anspruch 23, **dadurch gekennzeichnet, dass** als weiterer Schritt vorgesehen ist Bestimmen, ob die Interaktion der PH-Domäne mit ihren natürlichen Liganden inhibiert wird, bevorzugterweise die Interaktion der PH-Domäne von Cytohesin-1 mit Phosphatidylinositol-3,4,5-trisphosphat inhibiert wird.

27. Verfahren nach einem der Ansprüche 23 bis 26, **dadurch gekennzeichnet, dass** die Kandidaten-Substanz zur Herstellung eines Medikamentes verwendet wird.

28. Verwendung eines Guaninnukleotid-Austauschfaktors als Zielmolekül im Rahmen eines in vitro Selektions-Verfahrens.

29. Verfahren zur Identifizierung und Isolierung von Nukleinsäuren, die in der Lage sind, an ein Zielmolekül zu binden, **gekennzeichnet durch** die folgenden Schritte:
- Inkubieren des Zielmoleküls oder eines Teils davon mit einer Vielzahl von Nukleinsäuren, bevorzugterweise einer Nukleinsäurebibliothek, wobei die Nukleinsäuren verschiedene Sequenzen aufweisen,
- Selektieren und Isolieren jener Nukleinsäuren, die in der Lage sind, an das Zielmolekül oder einen Teil davon zu binden,
- optional Amplifizieren der isolierten Nukleinsäuren and Wiederholen der ersten beiden Schritte; und
- optional Bestimmen der Sequenz und/oder Bindungsspezifität der isolierten Nukleinsäuren,
**dadurch gekennzeichnet**, dass das Zielmolekül ein Guaninnukleotid-Austauschfaktor, bevorzugterweise ein solcher für ARF ist.

30. Verwendung einer Nukleinsäure nach einem der vorangehenden Ansprüchen, bevorzugterweise in einem Komplex mit einem Guaninnukleotid-Austauschfaktor, zum rationalen Design von Verbindungen, bevorzugterweise von Inhibitoren und bevorzugtererweisen von niedermolekularen Inhibitoren.


SEQUENZPROTOKOLL

<110> NascaCell GmbH

<120> Intrazelluläre Nukleinsäureinhibitoren kleiner Guaninnukleotid-
Austauschfaktoren

<130> N10004 EP

<140>
<141>

<160> 14

<170> PatentIn Ver. 2.1

<210> 1
<211> 94
<212> RNA
<213> Künstliche Sequenz

<220>
<223> Beschreibung der künstlichen Sequenz: Cytohesin
      bindende Nukleinsäuresequenz

<400> 1
gggagagaca agcuuggguc agggagggua gggauuuggu ugucccaugu ucgcagccgu 60
agaagagaaa gagaaguuaa uuaaggaucc ucag                              94

<210> 2
<211> 94
<212> RNA
<213> Künstliche Sequenz

<220>
<223> Beschreibung der künstlichen Sequenz: Cytohesin
      bindende Nukleinsäuresequenz

<400> 2
gggagagaca agcuuggguc ggguucgcua aguuggucua ccccucucga auauccguua 60
agaagagaaa gagaaguuaa uuaaggaucc ucag                              94

<210> 3
<211> 93
<212> RNA
<213> Künstliche Sequenz

<220>
<223> Beschreibung der künstlichen Sequenz: Cytohesin
      bindende Nukleinsäuresequenz

<400> 3
gggagagaca agcuuggguc ugcucuccau gggugcucuu gaaaggggaa gggaugugga 60
gaagagaaag agaaguuaau uaaggauccu cag                               93

<210> 4
<211> 94
<212> RNA
<213> Künstliche Sequenz

<220>
<223> Beschreibung der künstlichen Sequenz: Cytohesin
      bindende Nukleinsäuresequenz

<400> 4
gggagagaca agcuuggguc gauguuggca ggcugucguc acuggugggu cuaauucccg 60
agaagagaaa gagaaguuaa uuaaggaucc ucag                              94

<210> 5
<211> 93
<212> RNA
<213> Künstliche Sequenz

<220>
<223> Beschreibung der künstlichen Sequenz: Cytohesin
      bindende Nukleinsäuresequenz

<400> 5
gggagagaca agcuuggguc ugcccgcuua gaaaucccaa agugcgggaa cgguugcgaa 60
gaagagaaag agaaguuaau uaaggauccu cag                               93

<210> 6
<211> 95
<212> RNA
<213> Künstliche Sequenz

<220>
<223> Beschreibung der künstlichen Sequenz: Cytohesin
      bindende Nukleinsäuresequenz

<400> 6
gggagagaca agcuuggguc gggaagggug gaagugcugc uuggugcucu guccuuuuuu 60
aagaagagaa agagaaguua auuaaggauc cucag                             95

<210> 7
<211> 96
<212> RNA
<213> Künstliche Sequenz

<220>
<223> Beschreibung der künstlichen Sequenz: Cytohesin
      bindende Nukleinsäuresequenz

<400> 7
gggagagaca agcuuggguc gucauccgug uuuugucaug uuuugauggg guuuggggug 60
ugauaagaga aagagaaguu aauuaaggau ccucag                            96

<210> 8
<211> 94
<212> RNA
<213> Künstliche Sequenz

<220>
<223> Beschreibung der künstlichen Sequenz: Cytohesin
      bindende Nukleinsäuresequenz

<400> 8
gggagagaca agcuuggguc uguuguugga ccaguguuau uuuacgcgau ugugcgguug 60
ugaagagaaa gagaaguuaa uuaaggaucc ucag                              94

<210> 9
<211> 94
<212> RNA
<213> Künstliche Sequenz

<220>
<223> Beschreibung der künstlichen Sequenz: Cytohesin
      bindende Nukleinsäuresequenz

<400> 9
gggagagaca agcuuggguc uauuaugccu uuagcuagcg cauucugugg ggugggugga 60
agaagagaaa gagaaguuaa uuaaggaucc ucag                              94


<210> 10
<211> 94
<212> RNA
<213> Künstliche Sequenz

<220>
<223> Beschreibung der künstlichen Sequenz: Cytohesin
      bindende Nukleinsäuresequenz

<400> 10
gggagagaca agcuuggguc ugguuugaac gggggcgcau cagccaugcu auaaacucca 60
agaagagaaa gagaaguuaa uuaaggaucc ucag                              94


<210> 11
<211> 94
<212> RNA
<213> Künstliche Sequenz

<220>
<223> Beschreibung der künstlichen Sequenz:
      Randomisierte Nukleinsäuresequenz

<400> 11
gggagagaca agcuuggguc gugucugugu agaggacagg gcgagagucu gauugucugc 60
agaagagaaa gagaaguuaa uuaaggaucc ucag                              94


<210> 12
<211> 39
<212> DNA
<213> Künstliche Sequenz

<220>
<223> Beschreibung der künstlichen Sequenz: Primer

<400> 12
tctaatacga ctcactatag ggagagacaa gcttgggtc                         39


<210> 13
<211> 30
<212> DNA
<213> Künstliche Sequenz

<220>
<223> Beschreibung der künstlichen Sequenz: Primer

<400> 13
ctgaggatcc ttaattaact tctctttctc                                        30


<210> 14
<211> 99
<212> DNA
<213> Künstliche Sequenz

<220>
<223> Beschreibung der künstlichen Sequenz: Primer

<400> 14
ctatagggag agacaagctt gggtcnnnnn nnnnnnnnnn nnnnnnnnnn nnnnnnnnnn 60
nnnnnagaag agaaagagaa gttaattaag gatcctcag                          99